# EUROPÄISCHE PATENTSCHRIFT

(11) **EP 1 404 650 B1**
(45) Veröffentlichungstag und Bekanntmachung des Hinweises auf die Patenterteilung: **13.02.2008**
(21) Anmeldenummer: 02774016.6
(22) Anmeldetag: 11.05.2002
(51) Int. Cl.: C07C 275/54, C07C 323/42, C07D 211/58, C07D 213/74, C07D 213/40, C07D 295/13, C07D 285/14, C07D 319/18, C07D 207/09, C07D 209/08, C07D 401/04, C07D 209/14, C07D 317/58, A61P 3/10, A61K 31/17

(54) **CARBONSÄUREAMID SUBSTITUIERTE PHENYLHARNSTOFFDERIVATE, VERFAHREN ZU IHRER HERSTELLUNG ALS ARZNEIMITTEL**
CARBOXAMIDE-SUBSTITUTED PHENYLUREA DERIVATIVES AND METHOD FOR PRODUCTION THEREOF AS MEDICAMENTS
DERIVES DE PHENYLUREE SUBSTITUES PAR AMIDE D'ACIDE CARBOXYLIQUE, PROCEDE POUR LEUR PRODUCTION EN TANT QUE MEDICAMENTS

(30) Priorität: 25.05.2001 DE 10125567; 21.02.2002 DE 10207369
(43) Veröffentlichungstag der Anmeldung: 07.04.2004
(73) Patentinhaber: Sanofi-Aventis Deutschland GmbH, 65929 Frankfurt am Main (DE)
(72) Erfinder: DEFOSSA, Elisabeth, 65510 Idstein (DE); KLABUNDE, Thomas, 65929 Frankfurt (DE); BURGER, Hans-Joerg, 65719 Hofheim (DE); HERLING, Andreas, 65520 Bad Camberg (DE); VON ROEDERN, Erich, 65795 Hattersheim (DE); PEUKERT, Stefan, 60313 Frankfurt (DE); ENHSEN, Alfons, 64572 Büttelborn (DE); BAUER, Armin, 65843 Sulzbach (Taunus) (DE); NEISES, Berd, 77652 Offenburg (DE); WENDT, Karl, Ulrich, 60433 Frankfurt (DE)
(86) Internationale Anmeldenummer: PCT/EP2002/005205
(87) Internationale Veröffentlichungsnummer: WO 2002/096864

(56) Entgegenhaltungen:
- WO-A-00/71506
- WO-A-01/94300

## Beschreibung

Die Erfindung betrifft Carbonamid substituierte Phenylharnstoffderivate sowie deren physiologisch verträgliche Salze und physiologisch funktionelle Derivate.

Es sind bereits strukturähnliche Acylphenylharnstoffderivate als Insektizide im Stand der Technik beschriebe (EP 0 136 745, EP 0 167 197, DE 29 26 480, J. Agric. Food Chem. 1999, 47, 3416-3424).

WO 00/71506 offenbart Harnstoffderivate, die sich zur Behandlung von Typ II Diabetes eignen.

Der Erfindung lag die Aufgabe zugrunde, Verbindungen zur Verfügung zu stellen, die eine therapeutisch verwertbare Blutzucker senkende Wirkung entfalten.

Die Erfindung betrifft daher Verbindungen der Formel I, 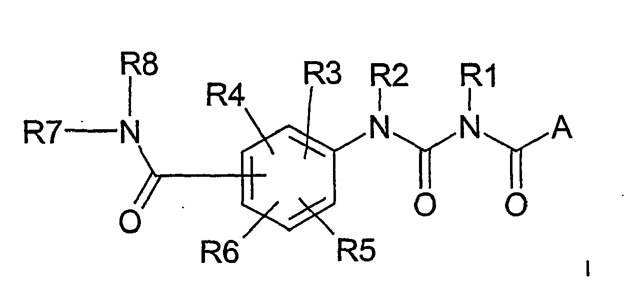 worin bedeuten
- A: Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, (C₀-C₆)-Alkylen-COOH, (C₀-C₆)-Alkylen-COO-(C₁-C₇)-alkyl, (C₀-C₆)-Alkylen-COO-(C₂-C₇)-alkenyl, CONH₂, CONH-(C₁-C₆)-Alkyl, CON-[(C₁-C₆)-Alkyl]₂, CONH-(C₃-C₆)-Cycloalkyl, (C₀-C₆)-Alkylen-NH₂, (C₀-C₆)-Alkylen-NH-(C₂-C₆)-alkyl, (C₀-C₆)-Alkylen-N-[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CONH₂ substituiert sein kann;
- R1, R2: unabhängig voneinander H, (C₁-C₆)-Alkyl, O-(C₁-C₆)-Alkyl, CO-(C₁-C₆)-Alkyl, COO-(C₁-C₆)-Alkyl;
- R3, R4, R5, R6: unabhängig voneinander H, F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, COOH, COO-(C₁-C₆)-Alkyl, CO-NH₂, CO-NH-(C₁-C₆)-Alkyl, CO-N-[(C₁-C₆)-Alkyl]₂, CO-NH-(C₃-C₇)-Cycloalkyl, NH₂, NH-(C₁-C₆)-Alkyl, N-[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CO-NH₂ substituiert sein kann;
- R7: H, (C₁-C₆)-Alkyl, CO(C₁-C₆)-Alkyl;
- R8: H, (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, N-[(C₁-C₆)-Alkyl]₂ NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkylen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl, Benzo[1,2,5]thiadiazolyl, Pyrrolidinyl, Morpholinyl sein kann und wobei der Arylrest ein oder mehrfach mit R9 substituiert sein kann;
- R9: F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie deren physiologisch verträgliche Salze.

Bevorzugt sind Verbindungen der Formel I, worin bedeuten
- A: Phenyl, wobei der Phenylrest bis zu dreifach substituiert sein kann mit F, Cl, Br;
- R1, R2: H;
- R3, R4, R5, R6: unabhängig voneinander H, F, Cl, Br, NO₂, O-(C₁-C₆)-Alkyl, (C₁-C₆)-Alkyl;
- R7: H, CH₃;
- R8: H, (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, N-[(C₁-C₆)-Alkyl]₂, NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkylen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl, Benzo[1,2,5]thiadiazoiyl, Pyrrolidinyl, Morpholinyl sein kann und wobei der Arylrest ein oder mehrfach mit R9 substituiert sein kann;
- R9: F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie deren physiologisch verträgliche Salze.

Besonders bevorzugt sind Verbindungen der Formel I, worin bedeuten
- A: Phenyl, wobei der Phenylrest bis zu dreifach substituiert sein kann mit F, Cl, Br;
- R1, R2: H;
- R3, R4, R5, R6: unabhängig voneinander H, F, Cl, Br, NO₂, O-(C₁-C₆)-Alkyl, (C₁-C₆)-Alkyl;
- R7: H, CH₃;
- R8: (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, N-[(C₁-C₆)-Alkyl]₂, NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkylen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl, Benzo[1,2,5]thiadiazolyl, Pyrrolidinyl, Morpholinyl sein kann und wobei der Arylrest ein oder mehrfach mit R9 substituiert sein kann;
- R9: F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie deren physiologisch verträgliche Salze.

Die Erfindung bezieht sich weiterhin auf die Verwendung der Verbindungen der Formel I 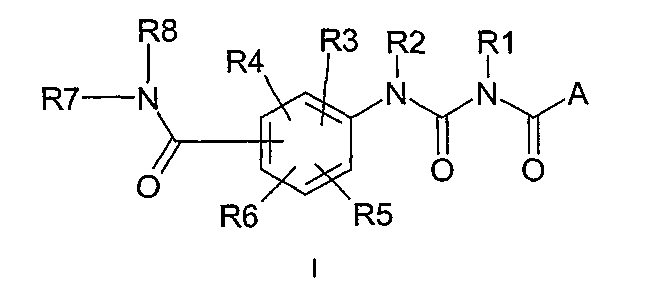 worin bedeuten
- A: Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, (C₀-C₆)-Alkylen-COOH, (C₀-C₆)-Alkylen-COO-(C₁-C₇)-alkyl, (C₀-C₆)-Alkylen-COO-(C₂-C₇)-alkenyl, CONH₂, CONH-(C₁-C₆)-Alkyl, CON-[(C₁-C₆)-Alkyl]₂, CONH-(C₃-C₆)-Cycloalkyl, (C₀-C₆)-Alkylen-NH₂, (C₀-C₆)-Alkylen-NH-(C₁-C₆)-alkyl, (C₀-C₆)-Alkylen-N-[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CONH₂ substituiert sein kann;
- R1, R2: unabhängig voneinander H, (C₁-C₆)-Alkyl, O-(C₁-C₆)-Alkyl, CO-(C₁-C₆)-Alkyl, COO-(C₁-C₆)-Alkyl;
- R3, R4, R5, R6: unabhängig voneinander H, F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)-Cycloalkyl, (C₃-C₇)Cycloalkyl-(C₁-C₄)-alkylen, COOH, COO-(C₁-C₆)-Alkyl, CO-NH₂, CO-NH-(C₁-C₆)Alkyl, CO-N-[(C₁-C₆)-Alkyl]₂, CO-NH-(C₃-C₇)-Cycloalkyl, NH₂, NH-(C₁-C₆)-Alkyl, N-[(C₁-C₆)Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CO-NH₂ substituiert sein kann;
- R7: H, (C₁-C₆)-Alkyl, CO(C₁-C₆)-Alkyl;
- R8: H, (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, oder N-[(C₁-C₆)-Alkyl]₂ , NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkylen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl, Benzo[1,2,5]thiadiazolyl, Pyrrolidinyl, Morpholinyl sein kann und wobei der Arylrest ein oder mehrfach mit R9 substituiert sein kann;
- R9: F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie derer physiologisch verträglichen Salze zur Herstellung eines Medikamentes zur Senkung des Blutzuckerspiegels und Behandlung von Typ II Diabetes.

Die Erfindung bezieht sich auf Verbindungen der Formel I, in Form ihrer Racemate, racemischen Mischungen und reinen Enantiomere sowie auf ihre Diastereomere und Mischungen davon.
Die Alkylreste in den Substituenten R1, R2, R3, R4, R5, R6, R7, R8, R9 und A können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Die Verbindung(en) der Formel (I) können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewähren spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wäßrigen oder nicht-wäßrigen Flüssigkeit; oder als eine Öl-in-Wasser oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete WirkstoffKonzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel 1 insbesonders zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Antidiabetika umfassen Insulin und Insulinderivate, wie z.B. Lantus^{®} oder HMR 1964, GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel 1 in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Bay 13-9952, BMS-201038, R-103757, verabreicht.

Bei einer Ausführungsform der Erfindung werden die Verbindungen der Formel I in Kombination mit Gallensäureresorptionsinhibitor, wie z.B. HMR 1453, verabreicht.

Bei einer Ausführungsform der Erfindung werden die Verbindungen der Formel I in Kombination mit einem CETP-Inhibitor, wie z.B. Bay 194789, verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer, wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen synthetase inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel l in Kombination mit Insulin verabreicht.

Bei einer Ausführungsform werden die Verbindungen der Formel l in Kombination mit einem Sulphonylharnstoff, wie z.B. Tolbutamid, Glibenclamid, Glipizid oder Gliclazid, verabreicht.

Bei einer Ausführungsform werden die Verbindungen der Formel l in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Gliclazid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormon, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen, wie z.B. Caromax^{®} verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß gemäß dem folgenden Reaktionsschema vorgegangen wird:

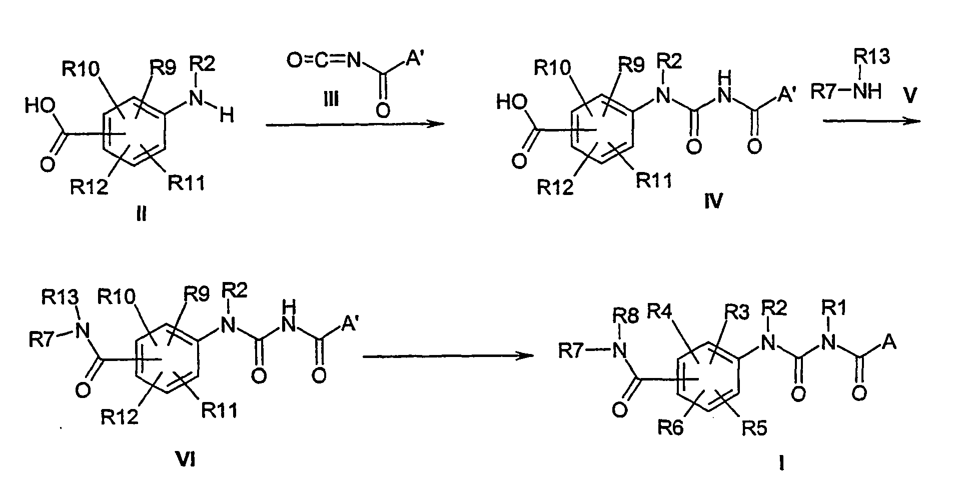

Dazu werden Verbindungen der allgemeinen Formel II, 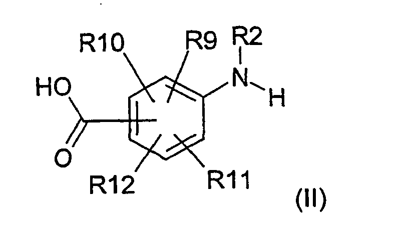 in denen
- R9, R10, R11, R12: unabhängig voneinander H, F, Cl, Br, O-(PG-1), CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-N-(PG-2)₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, COO-(PG-3), COO-(C₁-C₆)-Alkyl, CON-(PG-2)₂, CO-NH-(C₁-C₆)-Alkyl, CO-N-[(C₁-C₆)-Alkyl]₂, CO-NH-(C₃-C₇)-Cycloalkyl, N-(PG-2)₂, NH-(C₁-C₆)-Alkyl, N-[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, O-(PG-1), (C₁-C₆)-Alkyl, O-(C₁-C₆)-Alkyl, CF₃, OCF₃, COO-(PG-3), COO-(C₁-C₆)-Alkyl oder CON-(PG-2)₂ substituiert sein kann;
darstellt, worin R2 die oben beschriebene Bedeutung hat und
PG-1 eine allgemein bekannte Schutzgruppe für Alkohole, wie zum Beispiel Benzyl, Allyl, Tetrahydropyranyl oder Tetrahydrofuranyl;
PG-2 eine allgemein bekannte Schutzgruppe für Aminogruppen, wie zum Beispiel (C₁-C₆)-Alkylcarbonyl, (C₁-C₆)-Alkyloxycarbonyl oder (C₆-C₁₂)-Aryl-(C₁-C₄)-alkyloxycarbonyl, die entweder beide Wasserstoffe oder nur ein Wasserstoffatom der Aminogruppe ersetzt;
PG-3 eine allgemein bekannte Schutzgruppe für Ester, wie zum Beispiel (C₁-C₆)-Alkyl, Benzyl oder p-Methoxybenzyl;
darstellt; mit Isocyanaten der allgemeinen Formel III 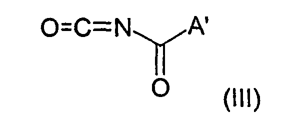 worin
- A': Phenyl, Naphthyl, wobei der Phenyl- oder Naphthylrest bis zu dreifach substituiert sein kann mit F, Cl, Br, O-PG-1, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-N-(PG-2)₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, (C₀-C₆)-Alkylen-COO-(PG-3), (C₀-C₆)Alkylen-COO-(C₁-C₇)-alkyl, (C₀-C₆)-Alkylen-COO-(C₂-C₇)-alkenyl, CO-N-(PG-2)₂, CO-NH-(C₁-C₆)-Alkyl, CO-N-[(C₁-C₆)-Alkyl]₂, CONH-(C₃-C₆)-Cycloalkyl, (C₀-C₆)-Alkylen-N-(PG-2)₂, (C₀-C₆)-Alkylen-NH-(C₁-C₆)-alkyl, (C₀-C₆)-Alkylen-N-[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, O-(PG-1), (C₁-C₆)-Alkyl, O-(C₁-C₆)-Alkyl, CF₃, OCF₃, COO-(PG-3), COO-(C₁-C₆)-Alkyl oder CO-N-(PG-2)₂ substituiert sein kann;
in denen PG-3, PG-2 und PG-1 die oben beschriebene Bedeutung haben,
in wasserfreien organischen Lösungsmitteln wie zum Beispiel Benzol, Toluol oder Acetonitril, unter Schutzgasatmosphäre bei Reaktionstemperaturen zwischen 10°C und der Siedetemperatur des eingesetzten Lösungsmittels zu Verbindungen der allgemeinen Formel IV 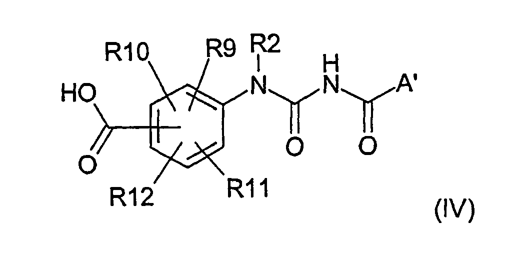 in denen R2, R9, R10, R11, R12, und A' die oben beschrieben Bedeutung haben, umgesetzt,
Verbindungen der allgemeinen Formel IV werden mit in der Peptid Synthese üblichen Kopplungsreagenzien, wie zum Beispiel Carbodiimide wie Dicyclohexylcarbodümid (DCC) oder Diisopropylcarbodiimid, Carbonyldiazole wie Carbonyldiimidazol und ähnliche Reagenzien, Propylphosphonsäureanhydride, O-((Cyano-(ethoxycarbonyl)-methylen)amino)-N,N,N',N'-tetramethyluronium tetrafluoroborat (TOTU) und viele andere, oder unter Ausbildung des Säurechlorides, zum Beispiel unter Verwendung von Thionylchlorid, mit Verbindungen der allgemeinen Formel V 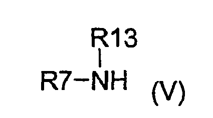 worin R7 die oben beschriebene Bedeutung hat und
- R13: (C₁-C₁₀)-Alkyl, wobei Alkyl bis zu 3 mal mit O-(PG-1), CF₃, CN, COO-(PG-3), COO-(C₁-C₆)-Alkyl, CO-N-(PG-2)₂, NH-(PG-2), NH-(C₁-C₆)Alkyl, N-[(C₁-C₆)-Alkyl]₂, substituiert sein kann;
Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl,
Benzo[1,2,5]thiadiazolyl, Pyrrolidinyl, Morpholinyl, wobei die Ringe jeweils ein oder mehrfach mit R14 substituiert sein können;
- R14: F, Cl, Br; O-(PG-1), NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COO-(PG-3), COO-(C₁-C₆)-Alkyl;
bedeuten
zu Verbindungen der allgemeinen Formel VI 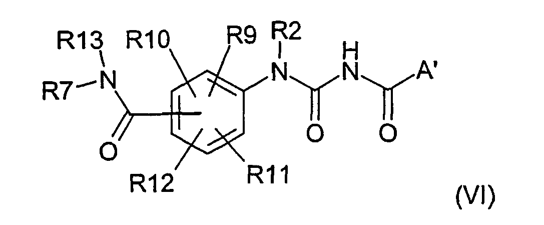 umgesetzt;
die Verbindungen der allgemeinen Formel VI können, falls R1 in Verbindungen der allgemeinen Formel I kein Wasserstoffatom darstellt, durch Umsetzung mit Verbindungen der allgemeinen Formel VII

R15-LG (VII)

worin
LG eine allgemein bekannte Austrittsgruppe, wie zum Beispiel Halogen, Arylsulfonyloxy oder Alkylsulfonyloxy;
und
- R15: (C₁-C₆)-Alkyl, O-(C₁-C₆)-Alkyl, CO-(C₁-C₆)-Alkyl, COO-(C₁-C₆)-Alkyl,
unter Verwendung einer Base, wie zum Beispiel 1,8-Diazabicyclo[5.4.0]undec-7-en, in organischen Lösungsmitteln, wie zum Beispiel Dichlormethan oder Acetonitril, zu Verbindungen der allgemeinen Formel VIII 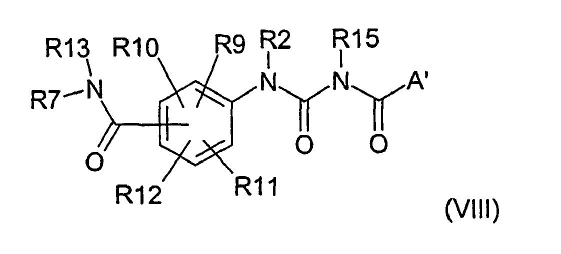 in denen R2, R7, R9, R10, R11, R12, R13, R15 und A' die oben beschrieben Bedeutung haben, alkyliert werden, und nach literaturbekannter Abspaltung einiger oder aller eventuell vorhandenen Schutzgruppen in den Resten R9, R10, R11, R12, R13, R14, und A' erhält man Verbindungen der allgemeinen Formel I. Die Überführung der Verbindungen der allgemeinen Formel I in deren Salze erfolgt durch Zugabe eines Äquivalentes der entsprechenden Säure oder Base in einem organischen Lösungsmittel wie zum Beispiel Acetonitril oder Dioxan oder in Wasser und durch anschließende Entfernung des Lösungsmittels.

Eine weitere Möglichkeit Verbindungen der allgemeinen Formel I, in denen R2 ein Wasserstoffatom darstellt, herzustellen ist im folgenden Schema dargestellt: 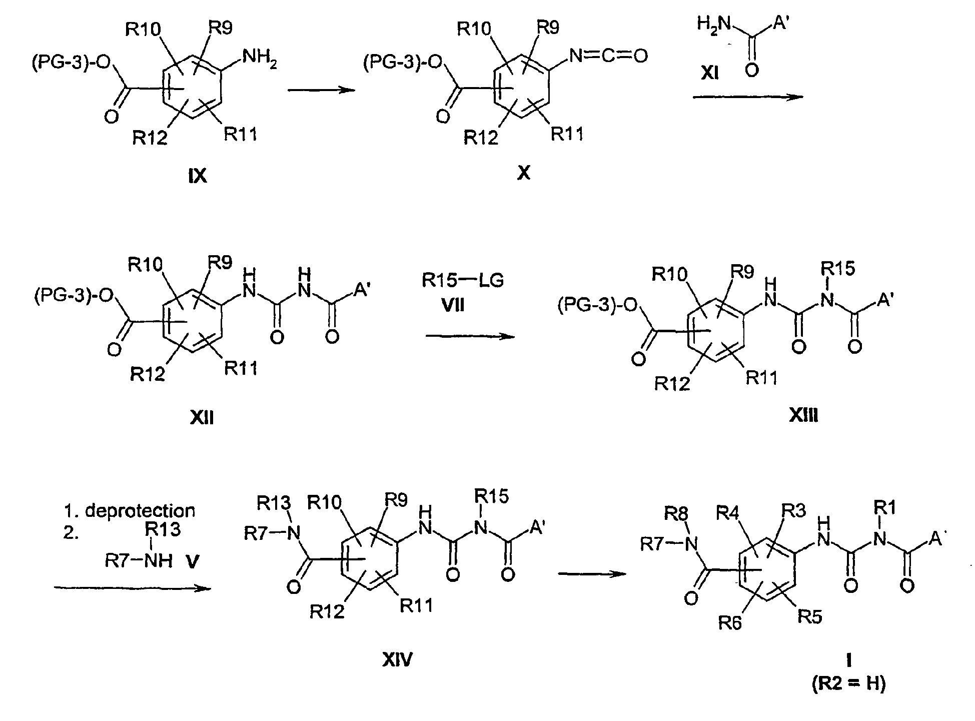 dabei werden Verbindungen der allgemeinen Formel XII, 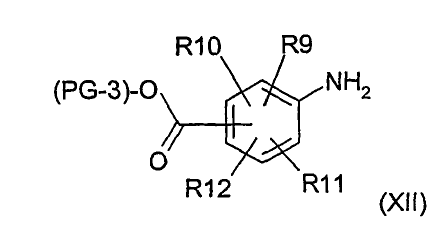 in denen R9, R10, R11, R12 und PG-3 die oben beschriebene Bedeutung haben, in Isocyanate der allgemeinen Formel X 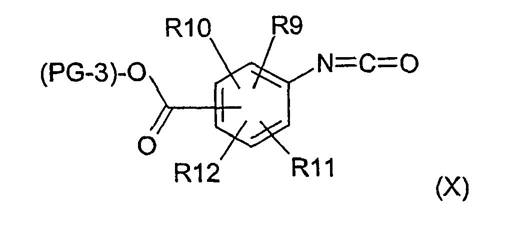 nach bekannten Methoden, wie zum Beispiel der Umsetzung mit Oxalylchlorid in organischen Lösungsmitteln, wie zum Beispiel 1,2-Dichlorethan oder Dichlormethan, bei Reaktionstemperaturen zwischen Raumtemperatur und dem Siedepunkt des Lösungsmittels umgewandelt,
die Isocyanate der allgemeinen Formel X bringt man mit Amiden der allgemeinen Formel XI 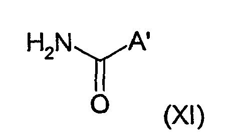 in denen A' die oben beschriebene Bedeutung hat,
zur Reaktion und erhält Verbindungen der allgemeinen Formel XII 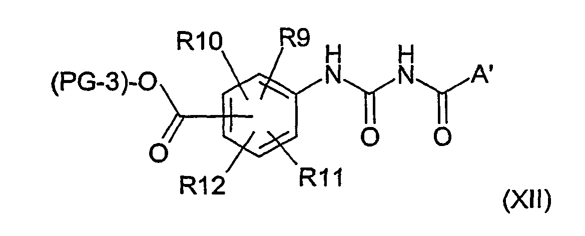 in denen R9, R10, R11, R12 und PG-3 die oben beschriebene Bedeutung haben, Verbindungen der allgemeinen Formel XII können, wenn R1 kein Wasserstoffatom darstellt, wie bereits oben beschrieben durch Alkylierung mit Verbindungen der allgemeinen Formel VII zu Verbindungen der allgemeinen Formel XIII, selektiver Entschützung der COO-(PG-3)-Gruppe und anschließende Amidkopplung mit Verbindungen der allgemeinen Formel V zu Verbindungen der allgemeinen Formel XIV und, falls notwendig, durch anschließende Abspaltung der Schutzgruppen in Verbindungen der allgemeinen Formel I überführt werden. Die Überführung der Verbindungen der allgemeinen Formel I in deren Salze erfolgt durch Zugabe eines Äquivalentes der entsprechenden Säure oder Base in einem organischen Lösungsmittel wie zum Beispiel Acetonitril oder Dioxan oder in Wasser und durch anschließende Entfernung des Lösungsmittels.

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

**Tabelle 1: Beispiele**

| | | | | | | | | | | | |
|---|---|---|---|---|---|---|---|---|---|---|---|
| 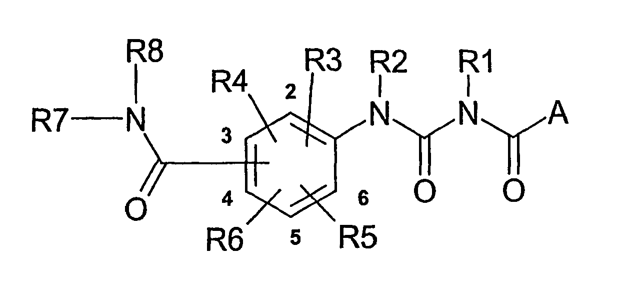 | | | | | | | | | | | |

| **Bsp.** | **A** | **R1*** | **R2** | **R3** | **R4** | **R6** | **R5** | **Amid**** | **R7** | **R8***** | **MS****** |
|---|---|---|---|---|---|---|---|---|---|---|---|
| **1** | Phenyl-2-Cl | H | H | 2-Cl | 3-H | 4-H | 6-H | 5 | H | 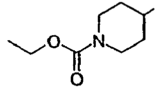 | ok |
| **2** | Phenyl-2-Cl | H | H | 2-H | 4-H | 5-H | 6-H | 3 | H | 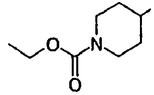 | ok |
| **3** | Phenyl-2-Cl | H | H | 2-H | 4-H | 5-H | 6-H | 3 | H | (CH₂)₅-OH | ok |
| **4** | Phenyl-2-Cl | H | H | 2-H | 4-H | 5-H | 6-H | 3 | H | (CH₂)₆-OH | ok |
| **5** | Phenyl-2-Cl | H | H | 2-H | 4-H | 5-H | 6-H | 3 | H | 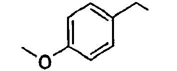 | ok |
| **6** | Phenyl-2-Cl | H | H | 2-H | 4-H | 5-H | 6-H | 3 | H | 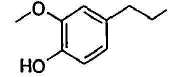 | ok |
| **7** | Phenyl-2-Cl | H | H | 2-H | 4-H | 5-H | 6-H | 3 | H | 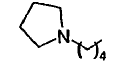 | ok |
| **8** | Phenyl-2-Cl | H | H | 2-H | 4-H | 5-H | 6-H | 3 | H | 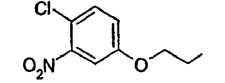 | ok |
| **9** | Phenyl-2-Cl | H | H | 2-H | 4-H | 5-H | 6-H | 3 | H | 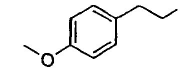 | ok |
| **10** | Phenyl-2-Cl | H | H | 2-H | 4-H | 5-H | 6-H | 3 | H | 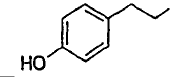 | ok |
| **11** | Phenyl-2-Cl | H | H | 2-H | 4-H | 5-H | 6-H | 3 | H | 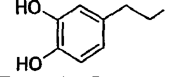 | ok |
| **12** | Phenyl-2-Cl | H | H | 2-H | 4-H | 5-H | 6-H | 3 | H | (CH₂)₃-COOtBu | ok |
| **13** | Phenyl-2-Cl | H | H | 2-H | 4-H | 5-H | 6-H | 3 | H | 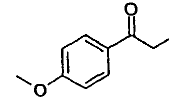 | ok |
| **14** | Phenyl-2-Cl | H | H | 2-H | 4-H | 5-H | 6-H | 3 | H | 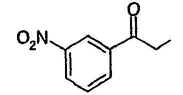 | ok |
| **15** | Phenyl-2-Cl | H | H | 2-H | 4-H | 5-H | 6-H | 3 | H | (CH₂)₅-CH₃ | ok |
| **16** | Phenyl-2-Cl | H | H | 2-H | 4-Cl | 5-H | 6-H | 3 | H | (CH₂)₅-OH | ok |
| **17** | Phenyl-2-Cl | H | H | 2-H | 4-Cl | 5-H | 6-H | 3 | H | (CH₂)₆-OH | ok |
| **18** | Phenyl-2-Cl | H | H | 2-H | 4-Cl | 5-H | 6-H | 3 | H | 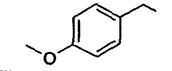 | ok |
| **19** | Phenyl-2-Cl | H | H | 2-H | 4-Cl | 5-H | 6-H | 3 | H | 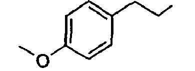 | ok |
| **20** | Phenyl-2-Cl | H | H | 2-H | 4-Cl | 5-H | 6-H | 3 | H | 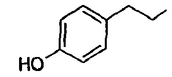 | ok |
| **21** | Phenyl-2-Cl | H | H | 2-H | 4-Cl | 5-H | 6-H | 3 | H | (CH₂)₅-CH₃ | ok |
| **22** | Phenyl-2-Cl | H | H | 2-CH₃ | 3-H | 4-H | 6-H | 5 | H | 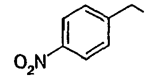 | ok |
| **23** | Phenyl-2-Cl | H | H | 2-CH₃ | 3-H | 4-H | 6-H | 5 | H | (CH₂)₅-OH | ok |
| **24** | Phenyl-2-Cl | H | H | 2-CH₃ | 3-H | 4-H | 6-H | 5 | H | (CH₂)₆-OH | ok |
| **25** | Phenyl-2-Cl | H | H | 2-CH₃ | 3-H | 4-H | 6-H | 5 | H | 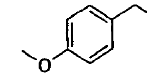 | ok |
| **26** | Phenyl-2-Cl | H | H | 2-CH₃ | 3-H | 4-H | 6-H | 5 | H | 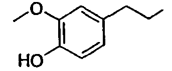 | ok |
| **27** | Phenyl-2-Ci | H | H | 2-CH₃ | 3-H | 4-H | 6-H | 5 | H | 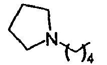 | ok |
| **28** | Phenyl-2-Cl | H | H | 2-CH₃ | 3-H | 4-H | 6-H | 5 | H | 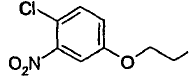 | ok |
| **29** | Phenyl-2-Cl | H | H | 2-CH₃ | 3-H | 4-H | 6-H | 5 | H | 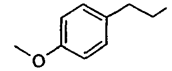 | ok |
| **30** | Phenyl-2-Cl | H | H | 2-CH₃ | 3-H | 4-H | 6-H | 5 | H | 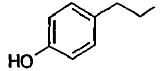 | ok |
| **31** | Phenyl-2-Cl | H | H | 2-CH₃ | 3-H | 4-H | 6-H | 5 | H | 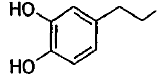 | ok |
| **32** | Phenyl-2-Cl | H | H | 2-CH₃ | 3-H | 4-H | 6-H | 5 | H | 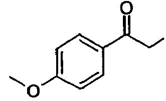 | ok |
| **33** | Phenyl-2-Cl | H | H | 2-CH₃ | 3-H | 4-H | 6-H | 5 | H | 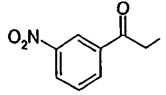 | ok |
| **34** | Phenyl-2-Cl | H | H | 2-CH₃ | 3-H | 4-H | 6-H | 5 | H | (CH₂)₅-CH₃ | ok |
| **35** | Phenyl-2-Cl | H | H | 2-OCH₃ | 3-H | 4-H | 6-H | 5 | H | 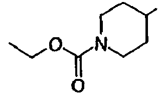 | ok |
| **36** | Phenyl-2-Cl | H | H | 2-OCH₃ | 3-H | 4-H | 6-H | 5 | H | 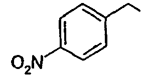 | ok |
| **37** | Phenyl-2-Cl | H | H | 2-OCH₃ | 3-H | 4-H | 6-H | 5 | H | (CH₂)₅-OH | ok |
| **38** | Phenyl-2-Cl | H | H | 2-OCH₃ | 3-H | 4-H | 6-H | 5 | H | (CH₂)₆-OH | ok |
| **39** | Phenyl-2-Cl | H | H | 2-OCH₃ | 3-H | 4-H | 6-H | 5 | H | 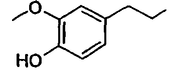 | ok |
| **40** | Phenyl-2-Cl | H | H | 2-OCH₃ | 3-H | 4-H | 6-H | 5 | H | 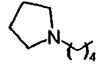 | ok |
| **41** | Phenyl-2-Cl | H | H | 2-OCH₃ | 3-H | 4-H | 6-H | 5 | H | 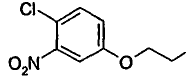 | ok |
| **42** | Phenyl-2-Cl | H | H | 2-OCH₃ | 3-H | 4-H | 6-H | 5 | H | 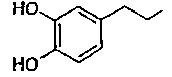 | ok |
| **43** | Phenyl-2-Cl | H | H | 2-OCH₃ | 3-H | 4-H | 6-H | 5 | H | (CH₂)₅-CH₃ | ok |
| **44** | Phenyl-2-Cl | H | H | 2-OCH₃ | 3-H | 4-H | 6-H | 5 | H | 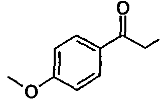 | ok |
| **45** | Phenyl-2-Cl | H | H | 2-OCH₃ | 3-H | 4-H | 6-H | 5 | H | 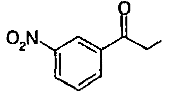 | ok |
| **46** | Phenyl-2-Cl | H | H | 2-OCH₃ | 3-H | 4-H | 6-H | 5 | H | 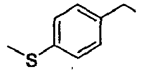 | ok |
| **47** | Phenyl-2-Cl | H | H | 2-H | 4-NO₂ | 5-H | 6-H | 3 | H | 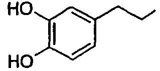 | ok |
| **48** | Phenyl-2-Cl | H | H | 2-H | 4-NO₂ | 5-H | 6-H | 3 | H | (CH₂)₅-OH | ok |
| **49** | Phenyl-2-Cl | H | H | 2-H | 4-NO₂ | 5-H | 6-H | 3 | H | (CH₂)₆-OH | ok |
| **50** | Phenyl-2-Cl | H | H | 2-H | 4-NO₂ | 5-H | 6-H | 3 | H | (CH₂)₅-CH₃ | ok |
| **51** | Phenyl-2-Cl | H | H | 2-H | 4-H | 5-H | 6-H | 3 | H | (CH₂)₃-COOH | ok |
| **52** | Phenyl-2-Cl | H | H | 2-OCH₃ | 3-H | 4-H | 6-H | 5 | H | (CH₂)₃-COOH | ok |
| **53** | Phenyl-2,6-Cl₂ | H | H | 2-OCH₃ | 3-H | 4-H | 6-H | 5 | H | 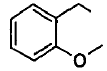 | ok |
| **54** | Phenyl-2,6-Cl₂ | H | H | 2-OCH₃ | 3-H | 4-H | 6-H | 5 | H | 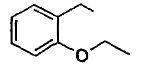 | ok |
| **55** | Phenyl-2,6-Cl₂ | H | H | 2-OCH₃ | 3-H | 4-H | 6-H | 5 | H | 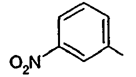 | ok |
| **56** | Phenyl-2-Cl | H | H | 2-OCH₃ | 3-H | 4-H | 6-H | 5 | H | 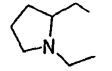 | ok |
| **57** | Phenyl-2-Cl | H | H | 2-OCH₃ | 3-H | 4-H | 6-H | 5 | H | 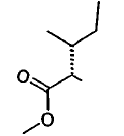 | ok |
| **58** | Phenyl-2-Cl | H | H | 2-OCH₃ | 3-H | 4-H | 6-H | 5 | H | 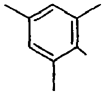 | ok |
| **59** | Phenyl-2-Cl | H | H | 2-OCH₃ | 3-H | 4-H | 6-H | 5 | H | 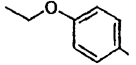 | ok |
| **60** | Phenyl-2-Cl | H | H | 2-OCH₃ | 3-H | 4-H | 6-H | 5 | H | 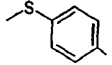 | ok |
| **61** | Phenyl-2-Cl | H | H | 2-OCH₃ | 3-H | 4-H | 6-H | 5 | H | 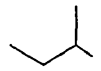 | ok |
| **62** | Phenyl-2-Cl | H | H | 2-OCH₃ | 3-H | 4-H | 6-H | 5 | H | 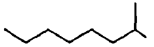 | ok |
| **63** | Phenyl-2-Cl | H | H | 2-OCH₃ | 3-H | 4-H | 6-H | 5 | H | 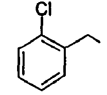 | ok |
| **64** | Phenyl-2-Cl | H | H | 2-OCH₃ | 3-H | 4-H | 6-H | 5 | H | 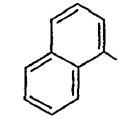 | ok |
| **65** | Phenyl-2-Cl | H | H | 2-OCH₃ | 3-H | 4-H | 6-H | 5 | H | 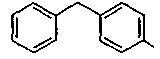 | ok |
| **66** | Phenyl-2-Cl | H | H | 2-OCH₃ | 3-H | 4-H | 6-H | 5 | H | 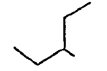 | ok |
| **67** | Phenyl-2-Cl | H | H | 2-OCH₃ | 3-H | 4-H | 6-H | 5 | H | 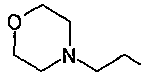 | ok |
| **68** | Phenyl-2-Cl | H | H | 2-OCH₃ | 3-H | 4-H | 6-H | 5 | H | 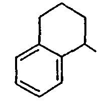 | ok |
| **69** | Phenyl-2-Cl | H | H | 2-OCH₃ | 3-H | 4-H | 6-H | 5 | H | 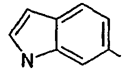 | ok |
| **70** | Phenyl-2-Cl | H | H | 2-H | 4-NO₂ | 5-H | 6-H | 3 | H | 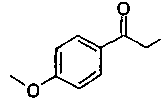 | ok |
| **71** | Phenyl-2-Cl | H | H | 2-H | 4-NO₂ | 5-H | 6-H | 3 | H | 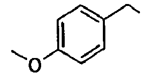 | ok |
| **72** | Phenyl-2-Cl | H | H | 2-OCH₃ | 3-H | 4-H | 6-H | 5 | H | 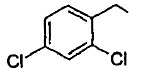 | ok |
| **73** | Phenyl-2-Cl | H | H | 2-OCH₃ | 3-H | 4-H | 6-H | 5 | H | 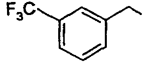 | ok |
| **74** | Phenyl-2-Cl | H | H | 2-OCH₃ | 3-H | 4-H | 6-H | 5 | H | CH₂-CF₃ | ok |
| **75** | Phenyl-2-Cl | H | H | 2-OCH₃ | 3-H | 4-H | 6-H | 5 | H | 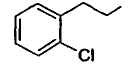 | ok |
| **76** | Phenyl-2-Cl | H | H | 2-OCH₃ | 3-H | 4-H | 6-H | 5 | H | 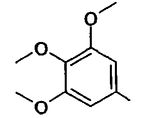 | ok |
| **77** | Phenyl-2-Cl | H | H | 2-OCH₃ | 3-H | 4-H | 6-H | 5 | H | 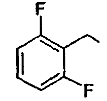 | ok |
| **78** | Phenyl-2-Cl | H | H | 2-OCH₃ | 3-H | 4-H | 6-H | 5 | H | 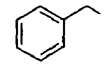 | ok |
| **79** | Phenyl-2-Cl | H | H | 2-OCH₃ | 3-H | 4-H | 6-H | 5 | H | (CH₂)₂-CH₃ | ok |
| **80** | Phenyl-2-Cl | H | H | 2-OCH₃ | 3-H | 4-H | 6-H | 5 | H | 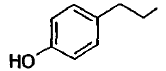 | ok |
| **81** | Phenyl-2-Cl | H | H | 2-OCH₃ | 3-H | 4-H | 6-H | 5 | H | 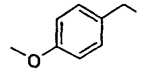 | ok |
| **82** | Phenyl-2-Cl | H | H | 2-OCH₃ | 3-H | 4-H | 6-H | 5 | H | 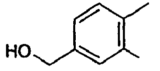 | ok |
| **83** | Phenyl-2-Cl | H | H | 2-OCH₃ | 3-H | 4-H | 6-H | 5 | H | 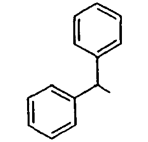 | ok |
| **84** | Phenyl-2-Cl | H | H | 2-OCH₃ | 3-H | 4-H | 6-H | 5 | H | 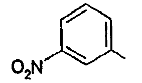 | ok |
| **85** | Phenyl-2-Cl | H | H | 2-OCH₃ | 3-H | 4-H | 6-H | 5 | H | 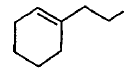 | ok |
| **86** | Phenyl-2-Cl | H | H | 2-OCH₃ | 3-H | 4-H | 6-H | 5 | H | 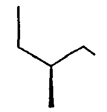 | ok |
| **87** | Phenyl-2-Cl | H | H | 2-OCH₃ | 3-H | 4-H | 6-H | 5 | H | 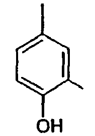 | ok |
| **88** | Phenyl-2-Cl | H | H | 2-OCH₃ | 3-H | 4-H | 6-H | 5 | H | 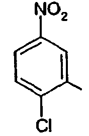 | ok |
| **89** | Phenyl-2-Cl | H | H | 2-OCH₃ | 3-H | 4-H | 6-H | 5 | H | 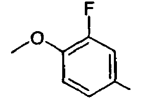 | ok |
| **90** | Phenyl-2-Cl | H | H | 2-OCH₃ | 3-H | 4-H | 6-H | 5 | H | 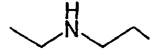 | ok |
| **91** | Phenyl-2-Cl | H | H | 2-OCH₃ | 3-H | 4-H | 6-H | 5 | H | 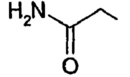 | ok |
| **92** | Phenyl-2-Cl | H | H | 2-OCH₃ | 3-H | 4-H | 6-H | 5 | H | 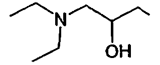 | ok |
| **93** | Phenyl-2-Cl | H | H | 2-OCH₃ | 3-H | 4-H | 6-H | 5 | H | 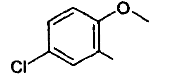 | ok |
| **94** | Phenyl-2,4-Cl₂ | Na | H | 2-OCH₃ | 3-H | 4-H | 6-H | 5 | H | 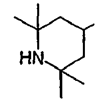 | ok |
| **95** | Phenyl-2,4-Cl₂ | Na | H | 2-OCH₃ | 3-H | 4-H | 6-H | 5 | H | 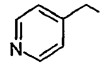 | ok |
| **96** | Phenyl-2,4-Cl₂ | Na | H | 2-OCH₃ | 3-H | 4-H | 6-H | 5 | H | 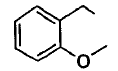 | ok |
| **97** | Phenyl-2,4-Cl₂ | Na | H | 2-OCH₃ | 3-H | 4-H | 6-H | 5 | H | 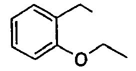 | ok |
| **98** | Phenyl-2,4-Cl₂ | Na | H | 2-OCH₃ | 3-H | 4-H | 6-H | 5 | H | 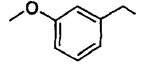 | ok |
| **99** | Phenyl-2,4-Cl₂ | Na | H | 2-OCH₃ | 3-H | 4-H | 6-H | 5 | H | 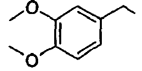 | ok |
| **100** | Phenyl-2,4-Cl₂ | Na | H | 2-OCH₃ | 3-H | 4-H | 6-H | 5 | H | 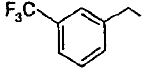 | ok |
| **101** | Phenyl-2,4-Cl₂ | Na | H | 2-OCH₃ | 3-H | 4-H | 6-H | 5 | H | 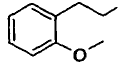 | ok |
| **102** | Phenyl-2,4-Cl₂ | Na | H | 2-OCH₃ | 3-H | 4-H | 6-H | 5 | H | 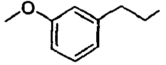 | ok |
| **103** | Phenyl-2,4-Cl₂ | Na | H | 2-OCH₃ | 3-H | 4-H | 6-H | 5 | H | 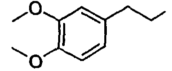 | ok |
| **104** | Phenyl-2,4-Cl₂ | Na | H | 2-OCH₃ | 3-H | 4-H | 6-H | 5 | H | 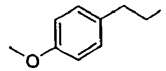 | ok |
| **105** | Phenyl-2,4-Cl₂ | Na | H | 2-OCH₃ | 3-H | 4-H | 6-H | 5 | H | (CH₂)₅-CN | ok |
| **106** | Phenyl-2,4-Cl₂ | Na | H | 2-OCH₃ | 3-H | 4-H | 6-H | 5 | H | 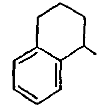 | ok |
| **107** | Phenyl-2,4-Cl₂ | Na | H | 2-OCH₃ | 3-H | 4-H | 6-H | 5 | H | 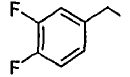 | ok |
| **108** | Phenyl-2,4-Cl₂ | Na | H | 2-OCH₃ | 3-H | 4-H | 6-H | 5 | H | 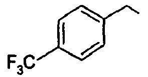 | ok |
| **109** | Phenyl-2,4-Cl₂ | Na | H | 2-OCH₃ | 3-H | 4-H | 6-H | 5 | H | 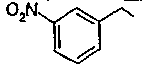 | ok |
| **110** | Phenyl-2,4-Cl₂ | Na | H | 2-OCH₃ | 3-H | 4-H | 6-H | 5 | H | 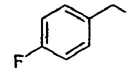 | ok |
| **111** | Phenyl-2,4-Cl₂ | Na | H | 2-OCH₃ | 3-H | 4-H | 6-H | 5 | H | 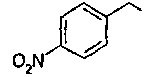 | ok |
| **112** | Phenyl-2,4-Cl₂ | Na | H | 2-OCH₃ | 3-H | 4-H | 6-H | 5 | H | 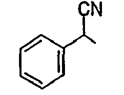 | ok |
| **113** | Phenyl-2,4-Cl₂ | Na | H | 2-OCH₃ | 3-H | 4-H | 6-H | 5 | H | 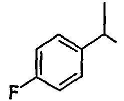 | ok |
| **114** | Phenyl-2,4-Cl₂ | Na | H | 2-OCH₃ | 3-H | 4-H | 6-H | 5 | H | 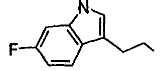 | ok |
| **115** | Phenyl-2,4,Cl₂ | Na | H | 2-OCH₃ | 3-H | 4-H | 6-H | 5 | H | 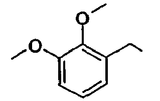 | ok |
| **116** | Phenyl-2,4-Cl₂ | Na | H | 2-OCH₃ | 3-H | 4-H | 6-H | 5 | H | 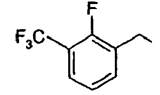 | ok |
| **117** | Phenyl-2,4-Cl₂ | Na | H | 2-OCH₃ | 3-H | 4-H | 6-H | 5 | H | 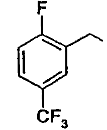 | ok |
| **118** | Phenyl-2,4-Cl₂ | Na | H | 2-OCH₃ | 3-H | 4-H | 6-H | 5 | H | 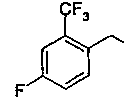 | ok |
| **119** | Phenyl-2,4-Cl₂ | Na | H | 2-OCH₃ | 3-H | 4-H | 6-H | 5 | H | 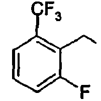 | ok |
| **120** | Phenyl-2,4-Cl₂ | Na | H | 2-OCH₃ | 3-H | 4-H | 6-H | 5 | H | 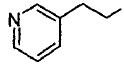 | ok |
| **121** | Phenyl-2,4-Cl₂ | Na | H | 2-OCH₃ | 3-H | 4-H | 6-H | 5 | H | 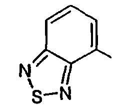 | ok |
| **122** | Phenyl-2,4-Cl₂ | Na | H | 2-OCH₃ | 3-H | 4-H | 6-H | 5 | H | 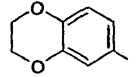 | ok |
| **123** | Phenyl-2,4-Cl₂ | Na | H | 2-OCH₃ | 3-H | 4-H | 6-H | 5 | H | 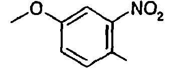 | ok |
| **124** | Phenyl-2,4-Cl₂ | Na | H | 2-OCH₃ | 3-H | 4-H | 6-H | 5 | H | 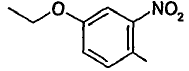 | ok |
| **125** | Phenyl-2,4-Cl₂ | Na | H | 2-OCH₃ | 3-H | 4-H | 6-H | 5 | H | 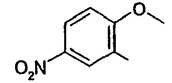 | ok |
| **126** | Phenyl-2,4-Cl₂ | Na | H | 2-OCH₃ | 3-H | 4-H | 6-H | 5 | H | 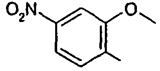 | ok |
| **127** | Phenyl-2,4-Cl₂ | Na | H | 2-OCH₃ | 3-H | 4-H | 6-H | 5 | H | 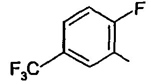 | ok |
| **128** | Phenyl-2,4-Cl₂ | Na | H | 2-OCH₃ | 3-H | 4-H | 6-H | 5 | H | 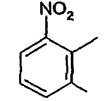 | ok |
| **129** | Phenyl-2,4-Cl₂ | Na | H | 2-OCH₃ | 3-H | 4-H | 6-H | 5 | H | 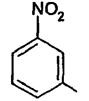 | ok |
| **130** | Phenyl-2,4-Cl₂ | Na | H | 2-OCH₃ | 3-H | 4-H | 6-H | 5 | H | 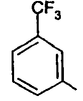 | ok |
| **131** | Phenyl-2,4-Cl₂ | Na | H | 2-OCH₃ | 3-H | 4-H | 6-H | 5 | H | 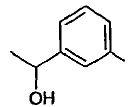 | ok |
| **132** | Phenyl-2,4-Cl₂ | Na | H | 2-OCH₃ | 3-H | 4-H | 6-H | 5 | H | 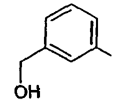 | ok |
| **133** | Phenyl-2,4-Cl₂ | Na | H | 2-OCH₃ | 3-H | 4-H | 6-H | 5 | H | 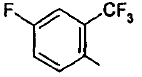 | ok |
| **134** | Phenyl-2,4-Cl₂ | Na | H | 2-OCH₃ | 3-H | 4-H | 6-H | 5 | H | 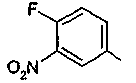 | ok |
| **135** | Phenyl-2,4-Cl₂ | Na | H | 2-OCH₃ | 3-H | 4-H | 6-H | 5 | H | 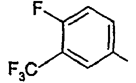 | ok |
| **136** | Phenyl-2,4-Cl₂ | Na | H | 2-OCH₃ | 3-H | 4-H | 6-H | 5 | H | 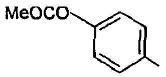 | ok |
| **137** | Phenyl-2,4-Cl₂ | Na | H | 2-OCH₃ | 3-H | 4-H | 6-H | 5 | H | 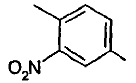 | ok |
| **138** | Phenyl-2,4-Cl₂ | Na | H | 2-OCH₃ | 3-H | 4-H | 6-H | 5 | H | 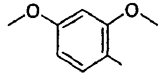 | ok |
| **139** | Phenyl-2,4-Cl₂ | Na | H | 2-OCH₃ | 3-H | 4-H | 6-H | 5 | H | 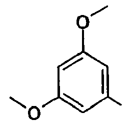 | ok |
| **140** | Phenyl-2,4-Cl₂ | Na | H | 2-OCH₃ | 3-H | 4-H | 6-H | 5 | H | 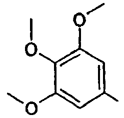 | ok |
| **141** | Phenyl-2,4-Cl₂ | Na | H | 2-OCH₃ | 3-H | 4-H | 6-H | 5 | H | 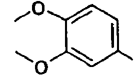 | ok |
| **142** | Phenyl-2,4-Cl₂ | Na | H | 2-OCH₃ | 3-H | 4-H | 6-H | 5 | H | 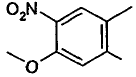 | ok |
| **143** | Phenyl-2,4-Cl₂ | Na | H | 2-OCH₃ | 3-H | 4-H | 6-H | 5 | H | 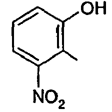 | ok |
| **144** | Phenyl-2,4-Cl₂ | Na | H | 2-OCH₃ | 3-H | 4-H | 6-H | 5 | H | 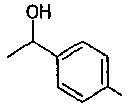 | ok |
| **145** | Phenyl-2,4-Cl₂ | Na | H | 2-OCH₃ | 3-H | 4-H | 6-H | 5 | H | 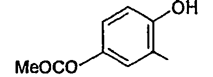 | ok |
| **146** | Phenyl-2,4-Cl₂ | Na | H | 2-OCH₃ | 3-H | 4-H | 6-H | 5 | H | 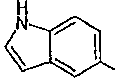 | ok |
| **147** | Phenyl-2,4-Cl₂ | Na | H | 2-OCH₃ | 3-H | 4-H | 6-H | 5 | H | 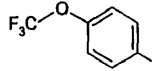 | ok |
| **148** | Phenyl-2,4-Cl₂ | Na | H | 2-OCH₃ | 3-H | 4-H | 6-H | 5 | H | 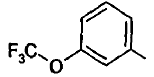 | ok |
| **149** | Phenyl-2,4-Cl₂ | Na | H | 2-OCH₃ | 3-H | 4-H | 6-H | 5 | H | 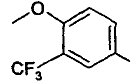 | ok |
| **150** | Phenyl-2,4-Cl₂ | Na | H | 2-OCH₃ | 3-H | 4-H | 6-H | 5 | H | 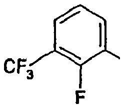 | ok |
| **151** | Phenyl-2,4-Cl₂ | Na | H | 2-OCH₃ | 3-H | 4-H | 6-H | 5 | H | 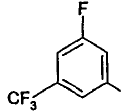 | ok |
| **152** | Phenyl-2,4-Cl₂ | Na | H | 2-OCH₃ | 3-H | 4-H | 6-H | 5 | H | 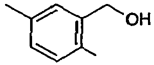 | ok |
| **153** | Phenyl-2-Cl | H | H | 2-H | 4-H | 5-NO₂ | 6-H | 3 | H | (CH₂)₅-OH | ok |
| **154** | Phenyl-2-Cl | H | H | 2-H | 4-H | 5-NO₂ | 6-H | 3 | H | (CH₂)₆-OH | ok |
| **155** | Phenyl-2-Cl | H | H | 2-H | 4-H | 5-NO₂ | 6-H | 3 | H | 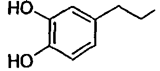 | ok |
| **156** | Phenyl-2-Cl | H | H | 2-H | 4-H | 5-NO₂ | 6-H | 3 | H | 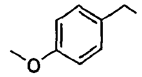 | ok |
| **157** | Phenyl-2-Cl | H | H | 2-F | 4-F | 5-F | 6-H | 3 | H | 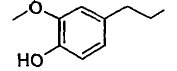 | ok |
| **158** | Phenyl-2-Cl | H | H | 2-F | 4-F | 5-F | 6-H | 3 | H | 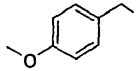 | ok |
| **159** | Phenyl-2-Cl | H | H | 2-F | 4-F | 5-F | 6-H | 3 | H | 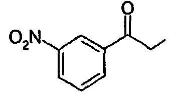 | ok |
| **160** | Phenyl-2-Cl | H | H | 2-F | 3-H | 4-H | 6-H | 5 | H | (CH₂)₅-OH | ok |
| **161** | Phenyl-2-Cl | H | H | 2-F | 3-H | 4-H | 6-H | 5 | H | 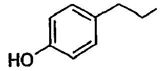 | ok |
| **162** | Phenyl-2-Cl | H | H | 2-F | 3-H | 4-H | 6-H | 5 | H | 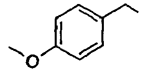 | ok |
| **163** | Phenyl-2-Cl | H | H | 2-F | 3-H | 4-H | 6-H | 5 | H | 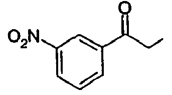 | ok |
| **164** | Phenyl-2,4-Cl₂ | H | H | 2-H | 4-H | 5-NO₂ | 6-H | 3 | H | (CH₂)₅-OH | ok |
| **165** | Phenyl-2,4-Cl₂ | H | H | 2-F | 4-F | 5-F | 6-H | 3 | H | (CH₂)₅-OH | ok |
| **166** | Phenyl-2,4-Cl₂ | H | H | 2-F | 4-F | 5-F | 6-H | 3 | H | (CH₂)₆-OH | ok |
| **167** | Phenyl-2,4-Cl₂ | H | H | 2-F | 3-H | 4-H | 6-H | 5 | H | (CH₂)₅-OH | ok |
| **168** | Phenyl-2,4-Cl₂ | H | H | 2-F | 3-H | 4-H | 6-H | 5 | H | (CH₂)₆-OH | ok |
| **169** | Phenyl-2,4-Cl₂ | H | H | 2-F | 3-H | 4-H | 6-H | 5 | H | 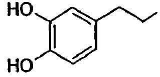 | ok |
| **170** | Phenyl-2,4-Cl₂ | H | H | 2-F | 3-H | 4-H | 6-H | 5 | H | 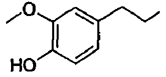 | ok |
| **171** | Phenyl-2,4-Cl₂ | H | H | 2-F | 3-H | 4-H | 6-H | 5 | H | 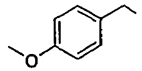 | ok |
| **172** | Phenyl-2,4-Cl₂ | H | H | 2-F | 3-H | 4-H | 6-H | 5 | H | 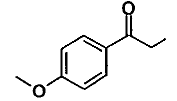 | ok |
| **173** | Phenyl-2,4-Cl₂ | H | H | 2-F | 3-H | 4-H | 6-H | 5 | H | 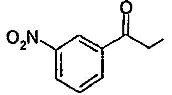 | ok |
| **174** | Phenyl-2,4-Cl₂ | H | H | 2-OCH₃ | 3-H | 4-H | 6-H | 5 | H | 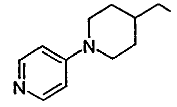 | ok |
| **175** | Phenyl-2,4-Cl₂ | H | H | 2-OCH₃ | 3-H | 4-H | 6-H | 5 | H | 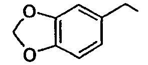 | ok |
| **176** | Phenyl-2,4-Cl₂ | H | H | 2-OCH₃ | 3-H | 4-H | 6-H | 5 | H | (CH₂)₂-COOH | ok |
| **177** | Phenyl-2,4-Cl₂ | H | H | 2-OCH₃ | 3-H | 4-H | 6-H | 5 | H | (CH₂)₃-COOH | ok |
| **178** | Phenyl-2-Cl | H | H | 2-OCH₃ | 3-H | 4-H | 6-H | 5 | H | 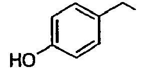 | ok |
| **179** | Phenyl-2,4-Cl₂ | H | H | 2-OCH₃ | 3-H | 4-H | 6-H | 5 | H | 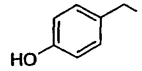 | ok |
| **180** | Phenyl-2,4-Cl₂ | H | H | 2-OCH₃ | 3-H | 4-H | 6-H | 5 | H | 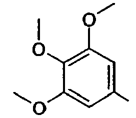 | ok |
| **181** | Phenyl-2,4-Cl₂ | H | H | 2-OCH₃ | 3-H | 4-H | 6-H | 5 | H | (CH₂)₄-COOH | ok |
| **182** | Phenyl-2,4-Cl₂ | H | H | 2-OCH₃ | 3-H | 4-H | 6-H | 5 | H | (CH₂)₅-COOH | ok |
| **183** | Phenyl-2-Cl | H | H | 2-OCH₃ | 3-H | 4-H | 6-H | 5 | H | 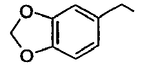 | ok |
| **184** | Phenyl-2-Cl | H | H | 2-OCH₃ | 3-H | 4-H | 6-H | 5 | H | (CH₂)₄-COOH | ok |
| **185** | Phenyl-2,4-Cl₂ | H | H | 2-OCH₃ | 3-H | 4-H | 6-H | 5 | H | 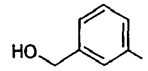 | ok |
| **186** | Phenyl-2,4-Cl₂ | H | H | 2-OCH₃ | 3-H | 4-H | 6-H | 5 | H | 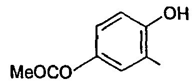 | ok |
| **187** | Phenyl-2-Cl | H | H | 2-NO₂ | 3-H | 5-H | 6-H | 4 | H | (CH₂)₅-OH | ok |
| **188** | Phenyl-2-Cl | H | H | 2-NO₂ | 3-H | 5-H | 6-H | 4 | H | (CH₂)₆-OH | ok |
| **189** | Phenyl-2-Cl | H | H | 2-NO₂ | 3-H | 5-H | 6-H | 4 | H | 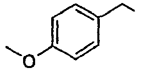 | ok |
| **190** | Phenyl-2-Cl | H | H | 2-NO₂ | 3-H | 5-H | 6-H | 4 | H | 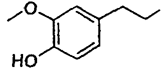 | ok |
| **191** | Phenyl-2-Cl | H | H | 2-NO₂ | 3-H | 5-H | 6-H | 4 | H | 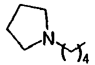 | ok |
| **192** | Phenyl-2-Cl | H | H | 2-NO₂ | 3-H | 5-H | 6-H | 4 | H | 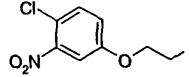 | ok |
| **193** | Phenyl-2-Cl | H | H | 2-NO₂ | 3-H | 5-H | 6-H | 4 | H | 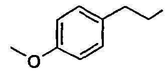 | ok |
| **194** | Phenyl-2-Cl | H | H | 2-NO₂ | 3-H | 5-H | 6-H | 4 | H | 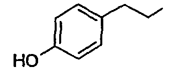 | ok |
| **195** | Phenyl-2-Cl | H | H | 2-NO₂ | 3-H | 5-H | 6-H | 4 | H | 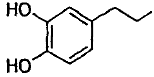 | ok |
| **196** | Phenyl-2-Cl | H | H | 2-NO₂ | 3-H | 5-H | 6-H | 4 | H | 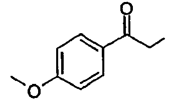 | ok |
| **197** | Phenyl-2-Cl | H | H | 2-H | 3-Cl | 5-H | 6-H | 4 | H | 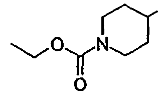 | ok |
| **198** | Phenyl-2-Cl | H | H | 2-H | 3-Cl | 5-H | 6-H | 4 | H | (CH₂)₅-OH | ok |
| **199** | Phenyl-2-Cl | H | H | 2-H | 3-Cl | 5-H | 6-H | 4 | H | 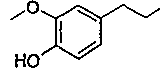 | ok |
| **200** | Phenyl-2-Cl | H | H | 2-H | 3-Cl | 5-H | 6-H | 4 | H | 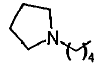 | ok |
| **201** | Phenyl-2-Cl | H | H | 2-H | 3-H | 5-H | 6-H | 4 | H | (CH₂)₅-OH | ok |
| **202** | Phenyl-2-Cl | H | H | 2-H | 3-H | 5-H | 6-H | 4 | H | (CH₂)₆-OH | ok |
| **203** | Phenyl-2-Cl | H | H | 2-H | 3-H | 5-H | 6-H | 4 | H | 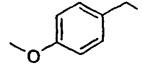 | ok |
| **204** | Phenyl-2-Cl | H | H | 2-H | 3-H | 5-H | 6-H | 4 | H | 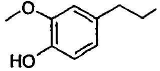 | ok |
| **205** | Phenyl-2-Cl | H | H | 2-H | 3-H | 5-H | 6-H | 4 | H | 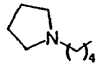 | ok |
| **206** | Phenyl-2-Cl | H | H | 2-H | 3-H | 5-H | 6-H | 4 | H | 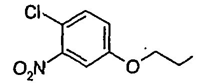 | ok |
| **207** | Phenyl-2-Cl | H | H | 2-H | 3-H | 5-H | 6-H | 4 | H | 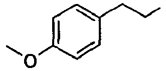 | ok |
| **208** | Phenyl-2-Cl | H | H | 2-H | 3-H | 5-H | 6-H | 4 | H | 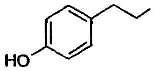 | ok |
| **209** | Phenyl-2-Cl | H | H | 2-H | 3-H | 5-H | 6-H | 4 | H | 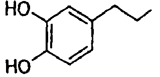 | ok |
| **210** | Phenyl-2-Cl | H | H | 2-H | 3-H | 5-H | 6-H | 4 | H | 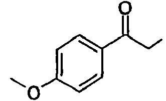 | ok |
| **211** | Phenyl-2-Cl | H | H | 2-Cl | 3-H | 5-H | 6-Cl | 4 | H | (CH₂)₆-OH | ok |
| **212** | Phenyl-2-Cl | H | H | 2-Cl | 3-H | 5-H | 6-Cl | 4 | H | (CH₂)₅-OH | ok |
| **213** | Phenyl-2-Cl | H | H | 2-Cl | 3-H | 5-OCH₃ | 6-H | 4 | H | (CH₂)₅-OH | ok |
| **214** | Phenyl-2-Cl | H | H | 2-Cl | 3-H | 5-OCH₃ | 6-H | 4 | H | (CH₂)₆-OH | ok |
| **215** | Phenyl-2-Cl | H | H | 2-Cl | 3-H | 5- OCH₃ | 6-H | 4 | H | 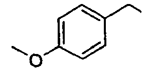 | ok |
| **216** | Phenyl-2-Cl | H | H | 2-Cl | 3-H | 5- OCH₃ | 6-H | 4 | H | 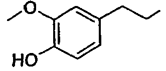 | ok |
| **217** | Phenyl-2-Cl | H | H | 2-Cl | 3-H | 5-OCH₃ | 6-H | 4 | H | 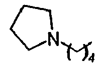 | ok |
| **218** | Phenyl-2-Cl | H | H | 2-Cl | 3-H | 5- OCH₃ | 6-H | 4 | H | 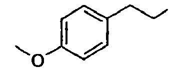 | ok |
| **219** | Phenyl-2-Cl | H | H | 2-Cl | 3-H | 5- OCH₃ | 6-H | 4 | H | 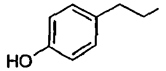 | ok |
| **220** | Phenyl-2-Cl | H | H | 2-NO₂ | 3-H | 5-H | 6-H | 4 | H | 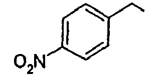 | ok |
| **221** | Phenyl-2-Cl | H | H | 2-H | 3-H | 5-H | 6-H | 4 | H | 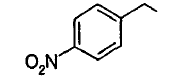 | ok |
| **222** | Phenyl-2-Cl | H | H | 2-H | 3-H | 5-H | 6-H | 4 | H | 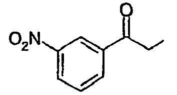 | ok |
| **223** | Phenyl-2-Cl | H | H | 2-OCH₃ | 3-H | 5-H | 6-H | 4 | H | (CH₂)₅-OH | ok |
| **224** | Phenyl-2-Cl | H | H | 2-OCH₃ | 3-H | 5-H | 6-H | 4 | H | (CH₂)₆-OH | ok |
| **225** | Phenyl-2-Cl | H | H | 2-OCH₃ | 3-H | 5-H | 6-H | 4 | H | 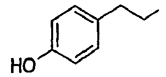 | ok |
| **226** | Phenyl-2-Cl | H | H | 2-OCH₃ | 3-H | 5-H | 6-H | 4 | H | 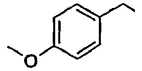 | ok |
| **227** | Phenyl-2-Cl | H | H | 2-Cl | 3-H | 5-H | 6-H | 4 | H | (CH₂)₅-OH | ok |
| **228** | Phenyl-2-Cl | H | H | 2-Cl | 3-H | 5-H | 6-H | 4 | H | (CH₂)₆-OH | ok |
| **229** | Phenyl-2-Cl | H | H | 2-Cl | 3-H | 5-H | 6-H | 4 | H | 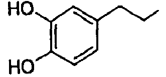 | ok |
| **230** | Phenyl-2-Cl | H | H | 2-Cl | 3-H | 5-H | 6-H | 4 | H | 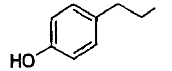 | ok |
| **231** | Phenyl-2-Cl | H | H | 2-Cl | 3-H | 5-H | 6-H | 4 | H | 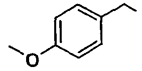 | ok |
| **232** | Phenyl-2-Cl | H | H | 2-F | 3-F | 5-F | 6-F | 4 | H | (CH₂)₅-OH | ok |
| **233** | Phenyl-2,4-Cl₂ | H | H | 2-OCH₃ | 3-H | 5-H | 6-H | 4 | H | (CH₂)₅-OH | ok |
| **234** | Phenyl-2,4-Cl₂ | H | H | 2-OCH₃ | 3-H | 5-H | 6-H | 4 | H | (CH₂)₆-OH | ok |
| **235** | Phenyl-2,4-Cl₂ | H | H | 2-OCH₃ | 3-H | 5-H | 6-H | 4 | H | 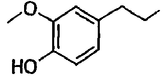 | ok |
| **236** | Phenyl-2,4-Cl₂ | H | H | 2-OCH₃ | 3-H | 5-H | 6-H | 4 | H | 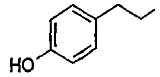 | ok |
| **237** | Phenyl-2,4-Cl₂ | H | H | 2-OCH₃ | 3-H | 5-H | 6-H | 4 | H | 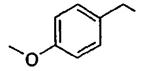 | ok |
| **238** | Phenyl-2,4-Cl₂ | H | H | 2-OCH₃ | 3-H | 5-H | 6-H | 4 | H | 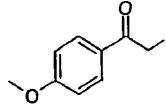 | ok |
| **239** | Phenyl-2,4-Cl₂ | H | H | 2-OCH₃ | 3-H | 5-H | 6-H | 4 | H | 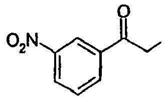 | ok |
| **240** | Phenyl-2,4-Cl₂ | H | H | 2-Cl | 3-H | 5-H | 6-H | 4 | H | (CH₂)₅-OH | ok |
| **241** | Phenyl-2,4-Cl₂ | H | H | 2-Cl | 3-H | 5-H | 6-H | 4 | H | (CH₂)₆-OH | ok |
| **242** | Phenyl-2,4-Cl₂ | H | H | 2-Cl | 3-H | 5-H | 6-H | 4 | H | 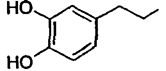 | ok |
| **243** | Phenyl-2,4-Cl₂ | H | H | 2-Cl | 3-H | 5-H | 6-H | 4 | H | 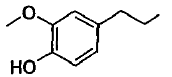 | ok |
| **244** | Phenyl-2,4-Cl₂, | H | H | 2-Cl | 3-H | 5-H | 6-H | 4 | H | 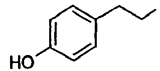 | ok |
| **245** | Phenyl-2,4-Cl₂ | H | H | 2-Cl | 3-H | 5-H | 6-H | 4 | H | 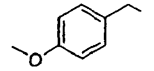 | ok |
| **246** | Phenyl-2,4-Cl₂ | H | H | 2-Cl | 3-H | 5-H | 6-H | 4 | H | 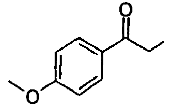 | ok |
| **247** | Phenyl-2,4-Cl₂ | H | H | 2-Cl | 3-H | 5-H | 6-H | 4 | H | 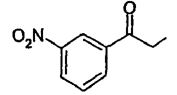 | ok |
| **248** | Phenyl-2,4-Cl₂ | H | H | 2-OH | 3-H | 5-H | 6-H | 4 | H | (CH₂)₆-OH | ok |
| | | | | | | | | | | | |
| **249** | Phenyl-2,4-Cl₂ | H | H | 2-NO₂ | 3-H | 5-H | 6-H | 4 | H | 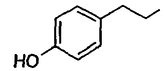 | ok |
| **250** | Phenyl-2-Cl | H | H | 2-OCH₃ | 3-H | 5-H | 6-H | 4 | H | 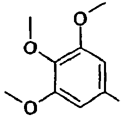 | ok |
| **251** | Phenyl-2,4-Cl₂ | H | H | 2-OCH₃ | 3-H | 5-H | 6-H | 4 | H | 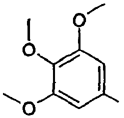 | ok |
| **252** | Phenyl-2-Cl | H | H | 2-Cl | 3-H | 5-H | 6-H | 4 | H | 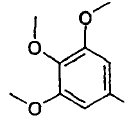 | ok |
| **253** | Phenyl-2,4-Cl₂ | H | H | 2-Cl | 3-H | 5-H | 6-H | 4 | H | 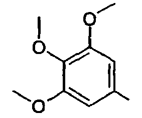 | ok |
| **254** | Phenyl-2,4-Cl₂ | H | H | 2-OCH₃ | 3-H | 4-H | 6-H | 5 | H | 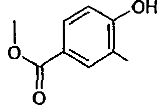 | ok |
| **255** | Phenyl-2-Cl-4-F | H | H | 2-OCH₃ | 3-H | 5-H | 6-H | 4 | H | H | ok |
| **256** | Phenyl-2,4-Cl₂ | H | H | 2-OCH₃ | 3-H | 5-H | 6-H | 4 | H | H | ok |
| **257** | Phenyl-2-Cl-4-F | H | H | 2-OCH₃ | 3-H | 5-H | 6-H | 4 | H | CH₃ | ok |
| **258** | Phenyl-2,4-Cl₂ | H | H | 2-OCH₃ | 3-H | 5-H | 6-H | 4 | H | CH₃ | ok |
| **259** | Phenyl-2-Cl-4-F | H | H | 2-OCH₃ | 3-H | 5-H | 6-H | 4 | H | (CH₂)₃-NHCOO-CH₂-Ph | ok |
| **260** | Phenyl-2-Cl-4,5-F₂ | H | H | 2-OCH₃ | 3-H | 5-H | 6-H | 4 | H | (CH₂)₃-NHCOO-CH₂-Ph | ok |
| **261** | Phenyl-2-Cl-4-F | H | H | 2-OCH₃ | 3-H | 5-H | 6-H | 4 | CH₃ | CH₃ | ok |
| **262** | Phenyl-2,4-Cl₂ | H | H | 2-OCH₃ | 3-H | 5-H | 6-H | 4 | CH₃ | CH₃ | ok |
| **263** | Phenyl-2-Cl-4,5-F₂ | H | H | 2-OCH₃ | 3-H | 5-H | 6-H | 4 | H | CH₃ | ok |
| **264** | Phenyl-2-Cl-4,5-F₂ | H | H | 2-OCH₃ | 3-H | 5-H | 6-H | 4 | CH₃ | CH₃ | ok |
| **265** | Phenyl-2-Cl-4,5-F₂ | H | H | 2-OCH₃ | 3-H | 5-H | 6-H | 4 | H | (CH₂)₂-NHCO-CH₃ | ok |
| **266** | Phenyl-2-Cl-4,5-F₂ | H | H | 2-OCH₃ | 3-H | 5-H | 6-H | 4 | H | (CH₂)₃-NH₂ TFA | ok |
| **267** | Phenyl-2-Cl-4,5-F₂ | H | H | 2-Cl | 3-H | 5-H | 6-H | 4 | H | CH₃ | ok |
| **268** | Phenyl-2-Cl-4,5-F₂ | H | H | 2-Cl | 3-H | 5-H | 6-H | 4 | CH₃ | CH₃ | ok |
| **269** | Phenyl-2-Cl-4,5-F₂ | H | H | 2-Cl | 3-H | 5-H | 6-H | 4 | H | H | ok |
| **270** | Phenyl-2-Cl-4,5-F₂ | H | H | 2-OCH₃ | 3-H | 5-H | 6-H | 4 | H | (CH₂)₃-N(CH₃)₂ TFA | ok |
| **271** | Phenyl-2-Cl-4,5-F₂ | H | H | 2-OCH₃ | 3-H | 5-H | 6-H | 4 | H | (CH₂)₂-N(CH₃)₂ TFA | ok |
| **272** | Phenyl-2,4-Cl₂ | H | H | 2-Cl | 3-H | 5-H | 6-H | 4 | H | (CH₂)₂-NHCOO-CH₂-CH=CH₂ | ok |
| **273** | Phenyl-2-Cl-4,5-F₂ | H | H | 2-OCH₃ | 3-H | 5-H | 6-H | 4 | H | (CH₂)₄-NH₂ TFA | ok |
| **274** | Phenyl-2,4-Cl₂ | H | H | 2-Cl | 3-H | 5-H | 6-H | 4 | H | (CH₂)₂-NH₂ TFA | ok |
| **275** | Phenyl-2-Cl-4-F | H | H | 2-OCH₃ | 3-H | 5-H | 6-H | 4 | H | (CH₂)₃-NH₂ TFA | ok |
| **276** | Phenyl-2-Cl-4,5-F₂ | H | H | 3-H | 4-H | 5-COOH | 6-H | 2 | H | CH₃ | ok |
| **277** | Phenyl-2-Cl-4,5-F₂ | H | H | 2-OCF₃ | 3-H | 5-H | 6-H | 4 | H | CH₃ | ok |
| **278** | Phenyl-2-Cl-4,5-F₂ | H | H | 2-OCF₃ | 3-H | 5-H | 6-H | 4 | CH₃ | CH₃ | ok |
| **279** | Phenyl-2-Cl-4,5-F₂ | H | H | 3-H | 4-H | 5-H | 6-H | 2 | H | H | ok |
| **280** | Phenyl-2-Cl-4,5-F₂ | H | H | 2-OCF₃ | 3-H | 5-H | 6-H | 4 | H | CH₂-COO-CH₃ | ok |
| **281** | Phenyl-2-Cl-4,5-F₂ | H | H | 2-OCF₃ | 3-H | 5-H | 6-H | 4 | CH₃ | CH₂-COO-CH₃ | ok |
| **282** | Phenyl-2-Cl-4,5-F₂ | H | H | 2-OCF₃ | 3-H | 5-H | 6-H | 4 | H | (CH₂)₂-COO-CH₃ | ok |
| **283** | Phenyl-2-Cl-4,5-F₂ | H | H | 2-OCF₃ | 3-H | 5-H | 6-H | 4 | H | (CH₂)₃-COO-CH₃ | ok |
| **284** | Phenyl-2-Cl-4,5-F₂ | H | H | 2-OCF₃ | 3-H | 5-H | 6-H | 4 | H | CH₂-COOH | ok |
| **285** | Phenyl-2-Cl-4,5-F₂ | H | H | 2-OCF₃ | 3-H | 5-H | 6-H | 4 | CH₃ | CH₂-COOH | ok |
| **286** | Phenyl-2-Cl-4,5-F₂ | H | H | 2-OCF₃ | 3-H | 5-H | 6-H | 4 | H | (CH₂)₂-COOH | ok |
| **287** | Phenyl-2-Cl-4,5-F₂ | H | H | 2-OCF₃ | 3-H | 5-H | 6-H | 4 | H | (CH₂)₃-COOH | ok |

| | | | | | | | | | | | |
|---|---|---|---|---|---|---|---|---|---|---|---|
| * "Na" bedeutet das Natriumsalz der entsprechenden Verbindung mit R1 = H ** In der Spalte "Amid" wird die Position der Carbonamidgruppe -(C=O)-N(R7) (R8) am Phenylrest angegeben. ***Sind bei R8 Strukturformeln angegeben, so erfolgt die Bindung von R8 an den Stickstoff über die verkürzt dargestellte Bindung **** Unter der Angabe "MS ist ok" wird verstanden, daß ein Massenspektrum gemessen wurde und in diesem der Molpeak (Molmasse + H⁺) nachgewiesen wurde | | | | | | | | | | | |

Die Verbindungen der Formel I zeichnen sich durch günstige Wirkungen auf den Zuckerstoffwechsel aus, sie senken insbesondere den Blutzuckerspiegel und sind zur Behandlung von Typ II Diabetes geeignet. Die Verbindungen können allein oder in Kombination mit weiteren Blutzucker senkenden Wirkstoffen (Antidiabetika) eingesetzt werden. Solche weiteren Blutzucker senkenden Wirkstoffe sind zum Beispiel Sulfonylharnstoffe (wie zum Beispiel Glimepirid, Glibenclamid, Gliclazid, Glibornurid,Gliquidon, Glisoxepid), Metformin, Tolbutamid, Glitazone (wie zum Beispiel Troglitazon, Rosiglitazon, Pioglitazon, Repaglinid), alpha-Glucosidase-Hemmer (wie zum Beispiel Acarbose, Miglitol) oder Insuline. Alle Antidiabetika, die in der Roten Liste 2001, Kapitel 12 genannt sind, können mit den erfindungsgemäßen Verbindungen der Formel I zur Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Die Verbindungen der Formel I eignen sich weiterhin zur Behandlung von Diabetischen Spätschäden, wie z.B. Nephropathie, Retinopathie, Neuropathie sowie Herzinfarkt, Myocardialem Infarkt, peripheren arteriellen Verschlußkrankheiten, Thrombosen, Arteriosklerose, Syndrom X, Obesitas, Entzündungen, Immunkrankheiten, Autoimmunkrankheiten, wie z.B. AIDS, Asthma, Osteoporose, Krebs, Psoriasis, Alzheimer, Schizophrenie und Infektionskrankheiten.

Die Wirksamkeit der Verbindungen wurde wie folgt getestet:

### Glykogenphophorylase a Aktivitätstest

Der Effekt von Verbindungen auf die Aktivität der aktiven Form der Glykogenphosphorylase (GPa) wurde in der umgekehrten Richtung, durch Verfolgen der Glykogensynthese aus Glukose-1-Phosphat an Hand der Bestimmung der Freisetzung von anorganischem Phosphat, gemessen. Alle Reaktionen wurden als Doppelbestimmungen in Mikrotiterplatten mit 96-Vertiefungen (Half Area Plates, Costar Nr. 3696) durchgeführt, wobei die Änderung der Absorption auf Grund der Bildung des Reaktionsprodukts bei der weiter unten spezifizierten Wellenlänge in einem Multiskan Ascent Elisa Reader (Lab Systems, Finnland) gemessen wurde.

Um die GPa Enzymaktivität in der umgekehrten Richtung zu messen, wurde die Umwandlung von Glukose-1-Phosphat in Glykogen und anorganisches Phosphat nach der allgemeinen Methode von Engers et al. (Engers HD, Shechosky S, Madsen NB, Can J Biochem 1970 Jul;48(7):746-754) mit folgenden Modifikationen gemessen: Humane Glykogenphosphorylase a (zum Beispiel mit 0,76 mg Protein / ml (Aventis Pharma Deutschland GmbH), gelöst in Pufferlösung E (25 mM β-Glyzerophosphat, pH 7,0, 1 mM EDTA und 1 mM Dithiotreitol) wurde mit Puffer T (50 mM Hepes, pH 7,0, 100 mM KCl, 2,5 mM EDTA, 2,5 mM MgCl₂·6H₂O) und Zusatz von 5 mg/ml Glykogen auf eine Konzentration von 10 µg Protein/ml verdünnt. Prüfsubstanzen wurden als 10 mM Lösung in DMSO zubereitet und auf 50 µM mit Pufferlösung T verdünnt. Zu 10 µl dieser Lösung wurden 10 µl 37,5 mM Glukose, gelöst in Pufferlösung T und 5 mg/mL Glykogen, sowie 10 µl einer Lösung von humaner Glykogenphosphorylase a (10 µg Protein/ml) und 20 µl Glukose-1-Phosphat, 2,5 mM zugegeben. Der basale Wert der Glykogenphosphorylase a Aktivität in Abwesenheit von Prüfsubstanz wurde durch Zugabe von 10 µl Pufferlösung T (0,1 % DMSO) bestimmt. Die Mischung wurde 40 Minuten bei Raumtemperatur inkubiert und das freigesetzte anorganische Phosphat mittels der allgemeinen Methode von Drueckes et al. (al (Drueckes P, Schinzel R, Palm D, Anal Biochem 1995 Sep 1;230(1):173-177) mit folgenden Modifikationen gemessen: 50 µl einer Stop-Lösung von 7,3 mM Ammoniummolybdat, 10,9 mM Zinkacetat, 3,6 % Askorbinsäure, 0,9 % SDS werden zu 50 µl der Enzymmischung gegeben. Nach 60 Minuten Inkubation bei 45 °C wurde die Absorption bei 820 nm gemessen. Zur Bestimmung der Hintergrundsabsorption wurde in einem separaten Ansatz die Stop-Lösung unmittelbar nach Zugabe der Glukose-1-Phosphatlösung zugegeben. Dieser Test wurde mit einer Konzentrationen von 10 µM der Prüfsubstanz durchgeführt, um die jeweilige Hemmung der Glykogenphosphorylase a in vitro durch die Prüfsubstanz zu bestimmen.

**Tabelle 2: Biologische Aktivität**

| Bsp. | % Hemmung bei 10 µM |
|---|---|
| 1 | 87 |
| 2 | 73 |
| 3 | 75 |
| 4 | 79 |
| 5 | 77 |
| 12 | 92 |
| 20 | 35 |
| 29 | 78 |
| 30 | 76 |
| 31 | 86 |
| 41 | 50 |
| 44 | 11 |
| 46 | 36 |
| 47 | 46 |
| 49 | 13 |
| 51 | 36 |
| 53 | 22 |
| 60 | 36 |
| 70 | 86 |
| 75 | 41 |
| 80 | 50 |
| 84 | 44 |
| 89 | 90 |
| 90 | 34 |
| 100 | 78 |
| 101 | 93 |
| 102 | 14 |
| 106 | 35 |
| 111 | 88 |
| 112 | 100 |
| 116 | 100 |
| 117 | 99 |
| 118 | 70 |
| 119 | 97 |
| 120 | 40 |
| 122 | 12 |
| 128 | 95 |
| 147 | 88 |
| 149 | 76 |

Aus der Tabelle ist abzulesen, daß die Verbindungen der Formel I die Aktivität der Glykogenphosphorylase a hemmen und dadurch zur Senkung des Blutzuckerspiegels gut geeignet sind.

Nachfolgend wird die Herstellung einiger Beispiele detailliert beschrieben, die übrigen Verbindungen der Formel I wurden analog erhalten:

### Experimenteller Teil:

### Beispiel 1:

### a) 2-Chlorbenzoylisocyanat

2-Chlorbenzamid wurde in Dichlormethan gelöst, mit 1,5 eq.Oxalylchlorid versetzt und 16 Stunden auf Rückfluss erhitzt. Das Reaktionsgemisch wurde am Hochvakuum eingeengt und ohne weitere Reinigung in Stufe b umgesetzt.

### b) 4-Chlor-3-[3-(2-chlor-benzoyl)-ureido]-benzoesäure

1 g (5,8 mmol) 3-Amino-4-chlor-benzoesäure wurden mit 0,75 g (5,8 mmol) Diisopropylethylamin und 1,06 g (5,8 mmol) 2-Chlorobenzoylisocyanat in 5 ml Dichlormethan versetzt und 12 Stunden bei Raumtemperatur zur Reaktion gebracht. Das Lösungsmittel wurde eingeengt, der Rückstand mit 5%iger Natriumhydrogencarbonat-Lösung versetzt, zweimal mit Diethylether extrahiert und die wässrige Phase mit HCl auf pH 3 gestellt. Der entstandene Niederschlag wurde abgesaugt.

### c) 4-{4-Chlor-3-[3-(2-chlor-benzoyl)-ureido]-benzoylamino}-piperidin-1-carbonsäureethylester

100 mg (0,28 mmol) 4-Chloro-3-[3-(2-chloro-benzoyl)-ureido]-benzoesäure, 93 mg (0,28 mmol) TOTU und 37 mg (0,28 mmol) Diisopropylethylamin wurden in 1 ml Dimethylformamid gekoppelt. Die Reaktionslösung wurde je einmal mit 5%iger Natriumhydrogencarbonat-Lösung und 10%iger Zitronensäure-Lösung gewaschen, die organische Phase getrocknet und eingeengt.

Die Beispiele 2-52 und 188-220 wurden analog Beispiel 1 synthetisiert.

### Beispiel 94:

### a) 4-[3-(2,4-Dichloro-benzoyl)-ureido]-3-methoxy-benzoesäure

Zu einer Lösung von 20 g (119,6 mmol) 4-Amino-3-methoxy-benzoesäure in 400 ml Acetonitril gab man 36,1 g (167,5 mmol) 2,4-Dichlorbenzoylisocyanat, das analog Beispiel 1 a hergestellt wurde. Es wurde 2 Stunden auf Rückfluss erhitzt und auf Raumtemperatur abgekühlt. Der Niederschlag wurde abgesaugt, mit Acetonitril und Methanol gewaschen, mit 5%iger Kaliumhydrogensulfat-Lösung verrührt, erneut abgesaugt und im Hochvakuum getrocknet. Man erhielt 44 g (96 %) des gewünschten Produktes.

### b) 4-[3-(2,4-Dichloro-benzoyl)-ureido]-3-methoxy-benzoyl chlorid

11,25 g (37,2 mmol) 4-[3-(2,4-Dichloro-benzoyl)-ureido]-3-methoxy-benzoesäure aus Stufe a wurde mit 150 ml Thionylchlorid 3 Stunden auf Rückfluss erhitzt und im Hochvakuum einrotiert. Der Rückstand wurde 2 mal mit Toluol versetzt und erneut im Hochvakuum eingeengt und man erhielt 10,88 g (27,09 mmol, 73%) Säurechlorid (Verlust durch überschäumen). Das so erhaltene Produkt wurde ohne weitere Reinigung in der nächste Stufe eingesetzt.

### c) 3-[3-(2,4-Dichlor-benzoyl)-ureido]-4-methoxy-N-(2,2,6,6-tetramethyl-piperidin-4-yl)-benzamid Natriumsalz

Eine Suspension von 157 mg (0,39 mmol) Säurechlorid aus Stufe b und 4 ml Dichlormethan gab man zu einer Lösung von 65 µl (0,8 mmol) Pyridin und 63 mg (0,4 mmol) 2,2,6,6-Tetramethyl-piperidin-4-ylamin in 2 ml Dichlormethan und brachte das Reaktiongemisch 16 Stunden bei Raumtemperatur zur Reaktion. Das reaktionsgemisch wurde mit 2,5 ml Acetonitril verdünnt, filtriert, mit 5 ml Acetonitril nachgewaschen und das Filtrat evaporiert. Nach Aufnahme des Rückstandes in einem Gemisch aus 2 n Natronlauge, Acetonitril und Dimethylformamid (1/2/2) fiel das Produkt aus.

Die Beispiele 95-152 wurden analog Beispiel 94 synthetisiert. Bei Bedarf wurden die Produkte über eine präparative HPLC/MS über reverse phase (Acetonitril/Wasser/TFA) gereinigt.

## Patentansprüche

1. Verbindungen der Formel I, 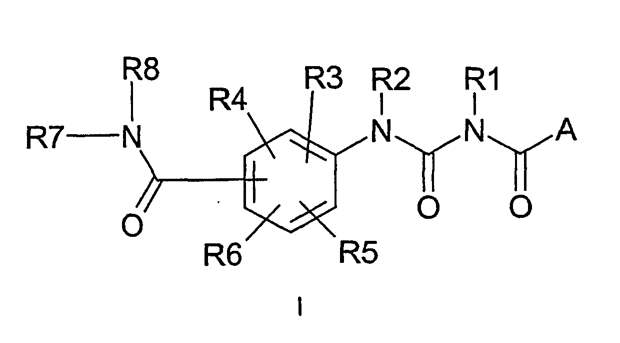 worin bedeuten
A Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)Cycloalkyl, (C₃-C₇)-Cycloalkyl-(C₁-C₄)-alkylen, (C₀-C₆)-Alkylen-COOH, (C₀-C₆)-Alkylen-COO-(C₁-C₇)-alkyl, (C₀-C₆)-Alkylen-COO-(C₂-C₇)-alkenyl, CONH₂, CONH-(C₁-C₆)-Alkyl, CON-[(C₁-C₆)-Alkyl]₂, CONH-(C₃-C₆)-Cycloalkyl, (C₀-C₆)-Alkylen-NH₂, (C₀-C₆)-Alkylen-NH-(C₂-C₆)-alkyl, (C₀-C₆)-Alkylen-N-[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CONH₂ substituiert sein kann;
R1, R2 unabhängig voneinander H, (C₁-C₆)-Alkyl, O-(C₁-C₆)-Alkyl, CO-(C₁-C₆)-Alkyl, COO-(C₁-C₆)-Alkyl;
R3, R4, R5, R6 unabhängig voneinander H, F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, COOH, COO-(C₁-C₆)-Alkyl, CO-NH₂, CO-NH-(C₁-C₆)-Alkyl, CO-N-[(C₁-C₆)-Alkyl]₂, CO-NH-(C₃-C₇)-Cycloalkyl, NH₂, NH-(C₁-C₆)-Alkyl, N-[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CO-NH₂ substituiert sein kann;
R7 H, (C₁-C₆)-Alkyl, CO(C₁-C₆)-Alkyl;
R8 H, (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, N-[(C₁-C₆)-Alkyl]₂ NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkylen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl, Benzo[1,2,5]thiadiazolyl, Pyrrolidinyl, Morpholinyl sein kann und wobei der Arylrest ein oder mehrfach mit R9 substituiert sein kann;
R9 F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie deren physiologisch verträgliche Salze.

2. Verbindungen der Formel I, gemäß Anspruch 1, **dadurch gekennzeichnet, daß** darin bedeuten
A Phenyl, wobei der Phenylrest bis zu dreifach substituiert sein kann mit F, Cl, Br;
R1, R2 H;
R3, R4, R5, R6 unabhängig voneinander H, F, Cl, Br, NO₂, O-(C₁-C₆)-Alkyl, (C₁-C₆)-Alkyl;
R7 H; CH₃
R8 H, (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, N-[(C₁-C₆)-Alkyl]₂ NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkylen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl, Benzo[1,2,5]thiadiazolyl, Pyrrolidinyl, Morpholinyl sein kann und wobei der Arylrest ein oder mehrfach mit R9 substituiert sein kann;
R9 F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie deren physiologisch verträgliche Salze.

3. Verbindungen der Formel I, gemäß Anspruch 1, **dadurch gekennzeichnet, daß** darin bedeuten
A Phenyl, wobei der Phenylrest bis zu dreifach substituiert sein kann mit F, Cl, Br;
R1, R2 H;
R3, R4, R5, R6 unabhängig voneinander H, F, Cl, Br, NO₂, O-(C₁-C₆)-Alkyl, (C₁-C₆)-Alkyl;
R7 H; CH₃
R8 (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, N-[(C₁-C₆)-Alkyl]₂ NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkyfen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl, Benzo[1,2,5]thiadiazolyl, Pyrrolidinyl, Morpholinyl sein kann und wobei der Arylrest ein oder mehrfach mit R9 substituiert sein kann;
R9 F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie deren physiologisch verträgliche Salze.

4. Arzneimittel enthaltend eine oder mehrere der Verbindungen gemäß einem oder mehreren der Ansprüche 1 bis 3.

5. Arzneimittel enthaltend eine oder mehrere der Verbindungen gemäß einem oder mehreren der Ansprüche 1 bis 3 und ein oder mehrere Blutzucker senkende Wirkstoffe.

6. Verwendung der Verbindungen gemäß einem oder mehreren der Ansprüche 1 bis 3 zur Herstellung eines Medikamentes zur Behandlung des Typ II Diabetes.

7. Verwendung der Verbindungen gemäß einem oder mehreren der Ansprüche 1 bis 3 zur Herstellung eines Medikamentes zur Blutzuckersenkung.

8. Verwendung der Verbindungen gemäß einem oder mehreren der Ansprüche 1 bis 3 in Kombination mit mindestens einem weiteren Blutzucker senkenden Wirkstoff zur Herstellung eines Medikamentes zur Behandlung des Typ II Diabetes.

9. Verwendung der Verbindungen gemäß einem oder mehreren der Ansprüche 1 bis 3 in Kombination mit mindestens einem weiteren Blutzucker senkenden Wirkstoff zur Herstellung eines Medikamentes zur Blutzuckersenkung.

10. Verfahren zur Herstellung eines Arzneimittels enthaltend eine oder mehrere der Verbindungen gemäß einem oder mehreren der Ansprüche 1 bis 3, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindung der Formel I 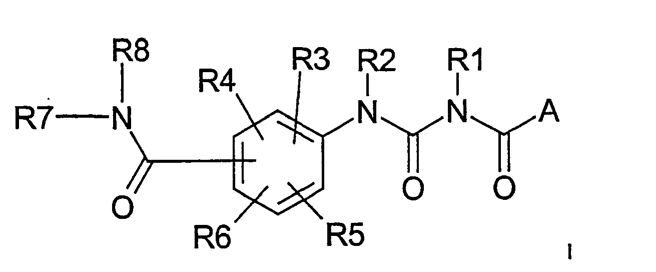 worin bedeuten
A Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, (C₀-C₆)-Alkylen-COOH, (C₀-C₆)-Alkylen-COO-(C₁-C₇)-alkyl, (C₀-C₆)-Alkylen-COO-(C₂-C₇)-alkenyl, CONH₂, CONH-(C₁-C₆)-Alkyl, CON-[(C₁-C₆)-Alkyl]₂, CONH-(C₃-C₆)-Cycloalkyl, (C₀-C₆)-Alkylen-NH₂, (C₀-C₆)-Alkylen-NH-(C₁-C₆)-alkyl, (C₀-C₆)-Alkylen-N-[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CONH₂ substituiert sein kann;
R1, R2 unabhängig voneinander H, (C₁-C₆)-Alkyl, O-(C₁-C₆)-Alkyl, CO-(C₁-C₆)-Alkyl, COO-(C₁-C₆)-Alkyl;
R3, R4, R5, R6 unabhängig voneinander H, F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆-Alkinyl, (C₃-C₇)-Cycloalkyl, (C₃-C₇)-Cycloalkyl-(C₁-C₄)-alkylen, COOH, COO-(C₁-C₆-Alkyl, CO-NH₂, CO-NH-(C₁-C₆)-Alkyl, CO-N-[(C₁-C₆-Alkyl]₂, CO-NH-(C₃-C₇)-Cycloalkyl, NH₂, NH-(C₁-C₆)-Alkyl, N-[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CO-NH₂ substituiert sein kann;
R7 H, (C₁-C₆)-Alkyl, CO(C₁-C₆)-Alkyl;
R8 H, (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, N-[(C₁-C₆)-Alkyl]₂ NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkylen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydrobenzo[1,4]dioxinyl, Benzo[1,2,5]thiadiazolyl, Pyrrolidinyl, Morpholinyl sein kann und wobei der Arylrest ein oder mehrfach mit R9 substituiert sein kann;
R9 F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie derer physiologisch verträglichen Salze zur Herstellung eines Medikamentes zur Senkung des Blutzuckerspiegels.

12. Verwendung der Verbindung der Formel I 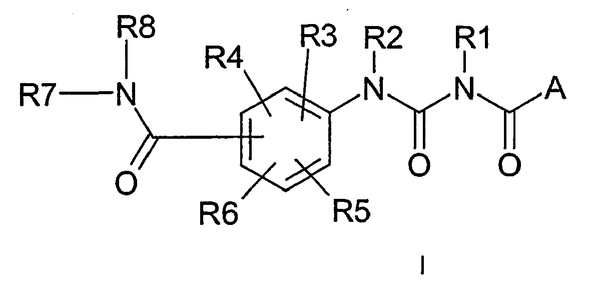 worin bedeuten
A Phenyl, Naphthyl, wobei der Phenyl- oder Naphthylrest bis zu dreifach substituiert sein kann mit F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, (C₀-C₆)-Alkylen-COOH, (C₀-C₆)-Alkylen-COO-(C₁-C₇)-alkyl, (C₀-C₆)-Alkylen-COO-(C₂-C₇)-alkenyl, CONH₂, CONH-(C₁-C₆)-Alkyl, CON-[(C₁-C₆)-Alkyl]₂, CONH-(C₃-C₆)-Cycloalkyl, (C₀-C₆)-Alkylen-NH₂, (C₀-C₆)-Alkylen-NH-(C₁-C₆)-alkyl, (C₀-C₆)-Alkylen-N-[(C₁-C₆)-alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CONH₂ substituiert sein kann;
R1, R2 unabhängig voneinander H, (C₁-C₆)-Alkyl, O-(C₁-C₆)-Alkyl, CO-(C₁-C₆)-Alkyl, COO-(C₁-C₆)-Alkyl;
R3, R4, R5, R6 unabhängig voneinander H, F, Cl, Br, OH, CF₃, NO₂, CN, OCF₃, O-(C₁-C₆)-Alkyl, O-(C₂-C₆)-Alkenyl, O-(C₂-C₆)-Alkinyl, S-(C₁-C₆)-Alkyl, S-(C₂-C₆)-Alkenyl, S-(C₂-C₆)-Alkinyl, SO-(C₁-C₆)-Alkyl, SO₂-(C₁-C₆)-Alkyl, SO₂-NH₂, (C₁-C₆)-Alkyl, (C₂-C₆)-Alkenyl, (C₂-C₆)-Alkinyl, (C₃-C₇)-Cycloalkyl, (C₃-C₇)-Cycloalkyl-(C₁-C₄)-alkylen, COOH, COO-(C₁-C₆)-Alkyl, CO-NH₂, CO-NH-(C₁-C₆)-Alkyl, CO-N-[(C₁-C₆)Alkyl]₂, CO-NH-(C₃-C₇)-Cycloalkyl, NH₂, NH-(C₁-C₆)-Alkyl, N-[(C₁-C₆)-Alkyl]₂, NH-CO-(C₁-C₆)-Alkyl, NH-CO-Phenyl, NH-SO₂-Phenyl, wobei der Phenylring bis zu zweifach mit F, Cl, CN, OH, (C₁-C₆)-Alkyl, O-(C₁-C₆)-Alkyl, CF₃, OCF₃, COOH, COO-(C₁-C₆)-Alkyl oder CO-NH₂ substituiert sein kann;
R7 H, (C₁-C₆)Alkyl, CO(C₁-C₆)-Alkyl;
R8 H, (C₁-C₁₀)-Alkyl, wobei Alkyl bis zu 3 mal mit OH, CF₃, CN, COOH, COO-(C₁-C₆)-Alkyl, CO-NH₂, NH₂, NH-(C₁-C₆)-Alkyl, N-[(C₁-C₆)-Alkyl]₂ NCO-(C₁-C₆)-alkyl, NCOO-(C₁-C₆)-alkyl, NCOO-(C₁-C₆)-alkenyl, NCOO-(C₁-C₆)-alkinyl oder NCOO-(C₁-C₄)-alkylen-(C₆-C₁₀)-aryl substituiert sein kann;
(CH₂)ₘ-Aryl, wobei m = 0-6 sein kann und Aryl gleich Phenyl, O-Phenyl, CO-Phenyl, Benzo[1,3]dioxolyl, Heterocycloalkyl, Pyridyl, Indolyl, Piperidinyl, Tetrahydronaphthyl, Naphthyl, 2,3-Dihydro-benzo[1,4]dioxinyl, Benzo[1,2,5]thiadiazolyl, Pyrrolidinyl, Morpholinyl sein kann und wobei der Arylrest ein oder mehrfach mit R9 substituiert sein kann;
R9 F, Cl, Br; OH, NO₂, CF₃, OCF₃, (C₁-C₆)-Alkyl, (C₁-C₆)-Alkyl-OH, O-(C₁-C₆)-Alkyl, S-(C₁-C₆)-Alkyl, (C₁-C₄)-Alkyl-Phenyl, COOH, COO-(C₁-C₆)-Alkyl;
sowie derer physiologisch verträglichen Salze zur Herstellung eines Medikamentes zur Behandlung von Typ II Diabetes.

## Claims

1. A compound of the formula I, 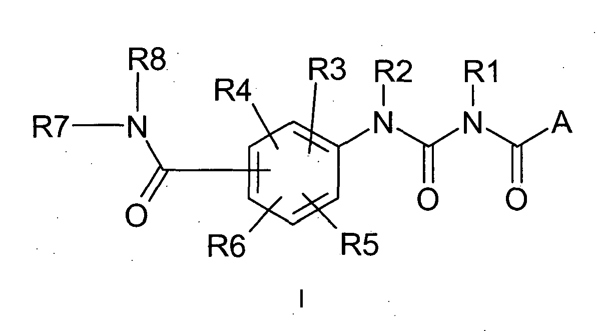 in which
A is phenyl, naphthyl, where the phenyl or naphthyl radical may be substituted up to three times by F, Cl, Br, OH, CF₃, NO₂, CN, OCF₃, O-(C₁-C₆)-alkyl, O-(C₂-C₆)-alkenyl, O-(C₂-C₆)-alkynyl, S-(C₁-C₆)-alkyl, S-(C₂-C₆)-alkenyl, S-(C₂-C₆)-alkynyl, SO-(C₁-C₆)-alkyl, SO₂-(C₁-C₆)-alkyl, SO₂-NH₂, (C₁-C₆)-alkyl, (C₂-C₆)-alkenyl, (C₂-C₆)-alkynyl, (C₃-C₇)-cycloalkyl, (C₃-C₇)-cycloalkyl-(C₁-C₄)-alkylene, (C₀-C₆)-alkylene-COOH, (C₀-C₆)-alkylene-COO-(C₁-C₇)-alkyl, (C₀-C₆)-alkylene-COO-(C₂-C₇)-alkenyl, CONH₂, CONH-(C₁-C₆)-alkyl, CON-[(C₁-C₆)-alkyl]₂, CONH-(C₃-C₆)-cycloalkyl, (C₀-C₆)-alkylene-NH₂, (C₀-C₆)-alkylene-NH-(C₂-C₆)-alkyl, (C₀-C₆)-alkylene-N-[(C₁-C₆)-alkyl]₂, NH-CO-(C₁-C₆)-alkyl, NH-CO-phenyl, NH-SO₂-phenyl, where the phenyl ring may be substituted up to twice by F, Cl, CN, OH, (C₁-C₆)-alkyl, O-(C₁-C₆)-alkyl, CF₃, OCF₃, COOH, COO-(C₁-C₆)-alkyl or CONH₂;
R1, R2 are, independently of one another, H, (C₁-C₆)-alkyl, O-(C₁-C₆)-alkyl, CO-(C₁-C₆)-alkyl, COO-(C₁-C₆)-alkyl;
R3, R4, R5, R6 are, independently of one another, H, F, Cl, Br, OH, CF₃, NO₂, CN, OCF₃, O-(C₁-C₆)-alkyl, O-(C₂-C₆)-alkenyl, O-(C₂-C₆)-alkynyl, S-(C₁-C₆)-alkyl, S-(C₂-C₆)-alkenyl, S-(C₂-C₆)-alkynyl, SO-(C₁-C₆)-alkyl, SO₂-(C₁-C₆)-alkyl, SO₂-NH₂, (C₁-C₆)-alkyl, (C₂-C₆)-alkenyl, (C₂-C₆)-alkynyl, (C₃-C₇)-cycloalkyl, (C₃-C₇)-cycloalkyl-(C₁-C₄)-alkylene, COOH, COO-(C₁-C₆)-alkyl, CO-NH₂, CO-NH-(C₁-C₆)-alkyl, CO-N-[(C₁-C₆)-alkyl]₂, CO-NH-(C₃-C₇)-cycloalkyl, NH₂, NH-(C₁-C₆)-alkyl, N-[(C₁-C₆)-alkyl]₂, NH-CO-(C₁-C₆)-alkyl, NH-CO-phenyl, NH-SO₂-phenyl, where the phenyl ring may be substituted up to twice by F, Cl, CN, OH, (C₁-C₆)-alkyl, O-(C₁-C₆)-alkyl, CF₃, OCF₃, COOH, COO-(C₁-C₆)-alkyl or CO-NH₂;
R7 is H, (C₁-C₆)-alkyl, CO(C₁-C₆)-alkyl;
R8 is H, (C₁-C₁₀)-alkyl, where alkyl may be substituted up to 3 times by OH, CF₃, CN, COOH, COO-(C₁-C₆)-alkyl, CO-NH₂, NH₂, NH-(C₁-C₆)-alkyl, N-[(C₁-C₆)-alkyl]₂, NCO-(C₁-C₆)-alkyl, NCOO-(C₁-C₆)-alkyl, NCOO-(C₁-C₆)-alkenyl, NCOO-(C₁-C₆)-alkynyl or NCOO-(C₁-C₄)-alkylene-(C₆-C₁₀)-aryl;
(CH₂)ₘ-aryl, where m can be 0-6, and aryl can be phenyl, O-phenyl, CO-phenyl, benzo[1,3]dioxolyl, heterocycloalkyl, pyridyl, indolyl, piperidinyl, tetrahydronaphthyl, naphthyl, 2,3-dihydro-benzo[1,4]dioxinyl, benzo[1,2,5]thiadiazolyl, pyrrolidinyl, morpholinyl, where the aryl radical may be substituted one or more times by R9;
R9 is F, Cl, Br; OH, NO₂, CF₃, OCF₃, (C₁-C₆)-alkyl, (C₁-C₆)-alkyl-OH, O-(C₁-C₆)-alkyl, S-(C₁-C₆)-alkyl, (C₁-C₄)-alkylphenyl, COOH, COO-(C₁-C₆)-alkyl;
and the physiologically tolerated salts thereof.

2. A compound of the formula I as claimed in claim 1, wherein
A is phenyl, where the phenyl radical may be substituted up to three times by F, Cl, Br;
R1, R2 are H;
R3, R4, R5, R6 are, independently of one another, H, F, Cl, Br, NO₂, O-(C₁-C₆)-alkyl, (C₁-C₆)-alkyl;
R7 is H, CH₃;
R8 is H, (C₁-C₁₀)-alkyl, where alkyl may be substituted up to 3 times by OH, CF₃, CN, COOH, COO-(C₁-C₆)-alkyl, CO-NH₂, NH₂, NH-(C₁-C₆)-alkyl, N-[(C₁-C₆)-alkyl]₂, NCO-(C₁-C₆)-alkyl, NCOO-(C₁-C₆)-alkyl, NCOO-(C₁-C₆)-alkenyl, NCOO-(C₁-C₆)-alkynyl or NCOO-(C₁-C₄)-alkylene-(C₆-C₁₀)-aryl;
(CH₂)ₘ-aryl, where m may be 0-6 and aryl may be phenyl, O-phenyl, CO-phenyl, benzo[1,3]dioxolyl, heterocycloalkyl, pyridyl, indolyl, piperidinyl, tetrahydronaphthyl, naphthyl, 2,3-dihydro-benzo[1,4]dioxinyl, benzo[1,2,5]thiadiazolyl, pyrrolidinyl, morpholinyl, and where the aryl radical may be substituted one or more times by R9;
R9 is F, Cl, Br; OH, NO₂, CF₃, OCF₃, (C₁-C₆)-alkyl, (C₁-C₆)-alkyl-OH, O-(C₁-C₆)-alkyl, S-(C₁-C₆)-alkyl, (C₁-C₄)-alkylphenyl, COOH, COO-(C₁-C₆)-alkyl;
and the physiologically tolerated salts thereof.

3. A compound of the formula I as claimed in claim 1, wherein
A is phenyl, where the phenyl radical may be substituted up to three times by F, Cl, Br;
R1, R2 are H;
R3, R4, R5, R6 are, independently of one another, H, F, Cl, Br, NO₂, O-(C₁-C₆)-alkyl, (C₁-C₆)-alkyl;
R7 is H, CH₃;
R8 is (C₁-C₁₀)-alkyl, where alkyl may be substituted up to 3 times by OH, CF₃, CN, COOH, COO-(C₁-C₆)-alkyl, CO-NH₂, NH₂, NH-(C₁-C₆)-alkyl, N-[(C₁-C₆)-alkyl]₂, NCO-(C₁-C₆)-alkyl, NCOO-(C₁-C₆)-alkyl, NCOO-(C₁-C₆)-alkenyl, NCOO-(C₁-C₆)-alkynyl or NCOO-(C₁-C₄)-alkylene-(C₆-C₁₀)-aryl;
(CH₂)ₘ-aryl, where m may be 0-6, and aryl may be phenyl, O-phenyl, CO-phenyl, benzo[1,3]dioxolyl, heterocycloalkyl, pyridyl, indolyl, piperidinyl, tetrahydronaphthyl, naphthyl, 2,3-dihydro-benzo[1,4]dioxinyl, benzo[1,2,5]thiadiazolyl, pyrrolidinyl, morpholinyl, and where the aryl radical may be substituted one or more times by R9;
R9 is F, Cl, Br; OH, NO₂, CF₃, OCF₃, (C₁-C₆)-alkyl, (C₁-C₆)-alkyl-OH, O-(C₁-C₆)-alkyl, S-(C₁-C₆)-alkyl, (C₁-C₄)-alkylphenyl, COOH, COO-(C₁-C₆)-alkyl;
and the physiologically tolerated salts thereof.

4. A medicament comprising one or more of the compounds as claimed in one or more of claims 1 to 3.

5. A medicament comprising one or more of the compounds as claimed in one or more of claims 1 to 3 and one or more blood glucose-lowering active ingredients.

6. The use of the compounds as claimed in one or more of claims 1 to 3 for producing a medicament for the treatment of type II diabetes.

7. The use of the compounds as claimed in one or more of claims 1 to 3 for producing a medicament for lowering blood glucose.

8. The use of the compounds as claimed in one or more of claims 1 to 3 in combination with at least one other blood glucose-lowering active ingredient for producing a medicament for the treatment of type II diabetes.

9. The use of the compounds as claimed in one or more of claims 1 to 3 in combination with at least one other blood glucose-lowering active ingredient for producing a medicament for lowering blood glucose.

10. A process for producing a medicament comprising one or more of the compounds as claimed in one or more of claims 1 to 3, which comprises mixing the active ingredient with a pharmaceutically suitable carrier and converting this mixture into a form suitable for administration.

11. The use of the compound of the formula I 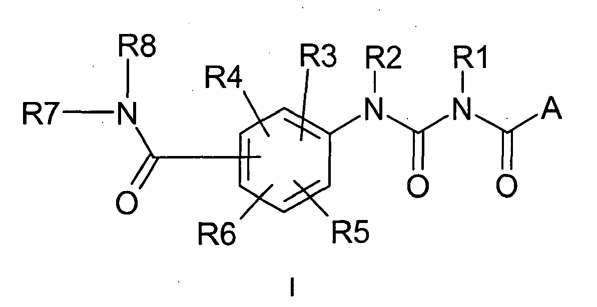 in which
A is phenyl, naphthyl, where the phenyl or naphthyl radical may be substituted up to three times by F, Cl, Br, OH, CF₃, NO₂, CN, OCF₃, O-(C₁-C₆)-alkyl, O-(C₂-C₆)-alkenyl, O-(C₂-C₆)-alkynyl, S-(C₁-C₆)-alkyl, S-(C₂-C₆)-alkenyl, S-(C₂-C₆)-alkynyl, SO-(C₁-C₆)-alkyl, SO₂-(C₁-C₆)-alkyl, SO₂-NH₂, (C₁-C₆)-alkyl, (C₂-C₆)-alkenyl, (C₂-C₆)-alkynyl, (C₃-C₇)-cycloalkyl, (C₃-C₇)-cycloalkyl-(C₁-C₄)-alkylene, (C₀-C₆)-alkylene-COOH, (C₀-C₆)-alkylene-COO-(C₁-C₇)-alkyl, (C₀-C₆)-alkylene-COO-(C₂-C₇)-alkenyl, CONH₂, CONH-(C₁-C₆)-alkyl, CON-[(C₁-C₆)-alkyl]₂; CON H-(C₃-C₆)-cycloalkyl, (C₀-C₆)-alkylene-NH₂, (C₀-C₆)-alkylene-NH-(C₁-C₆)-alkyl, (C₀-C₆)-alkylene-N-[(C₁-C₆)-alkyl]₂, NH-CO-(C₁-C₆)-alkyl, NH-CO-phenyl, NH-SO₂-phenyl, where the phenyl ring may be substituted up to twice by F, Cl, CN, OH, (C₁-C₆)-alkyl, O-(C₁-C₆)-alkyl, CF₃, OCF₃, COOH, COO-(C₁-C₆)-alkyl or CONH₂;
R1, R2 are, independently of one another, H, (C₁-C₆)-alkyl, O-(C₁-C₆)-alkyl, CO-(C₁-C₆)-alkyl, COO-(C₁-C₆)-alkyl;
R3, R4, R5, R6 are, independently of one another, H, F, Cl, Br, OH, CF₃, NO₂, CN, OCF₃, O-(C₁-C₆)-alkyl, O-(C₂-C₆)-alkenyl, O-(C₂-C₆)-alkynyl, S-(C₁-C₆)-alkyl, S-(C₂-C₆)-alkenyl, S-(C₂-C₆)-alkynyl, SO-(C₁-C₆)-alkyl, SO₂-(C₁-C₆)-alkyl, SO₂-NH₂, (C₁-C₆)-alkyl, (C₂-C₆)-alkenyl, (C₂-C₆)-alkynyl, (C₃-C₇)-cycloalkyl, (C₃-C₇)-cycoalkyl-(C₁-C₄)-alkylene, COOH, COO-(C₁-C₆)-alkyl, CO-NH₂, CO-NH-(C₁-C₆)-alkyl, CO-N-[(C₁-C₆)-alkyl]₂, CO-NH-(C₃-C₇)-cycloalkyl, NH₂, NH-(C₁-C₆)-alkyl, N-[(C₁-C₆)-alkyl]₂, NH-CO-(C₁-C₆)-alkyl, NH-CO-phenyl, NH-SO₂-phenyl, where the phenyl ring may be substituted up to twice by F, Cl, CN, OH, (C₁-C₆)-alkyl, O-(C₁-C₆)-alkyl, CF₃, OCF₃, COOH, COO-(C₁-C₆)-alkyl or CO-NH₂;
R7 is H, (C₁-C₆)-alkyl, CO(C₁-C₆)-alkyl;
R8 is H, (C₁-C₁₀)-alkyl, where alkyl may be substituted up to 3 times by OH, CF₃, CN, COOH, COO-(C₁-C₆)-alkyl, CO-NH₂, NH₂, NH-(C₁-C₆)-alkyl, N-[(C₁-C₆)-alkyl]₂, NCO-(C₁-C₆)-alkyl, NCOO-(C₁-C₆)-alkyl, NCOO-(C₁-C₆)-alkenyl, NCOO-(C₁-C₆)-alkynyl or NCOO-(C₁-C₄)-alkylene-(C₆-C₁₀)-aryl;
(CH₂)ₘ-aryl, where m can be 0-6, and aryl can be phenyl, O-phenyl, CO-phenyl, benzo[1,3]dioxolyl, heterocycloalkyl, pyridyl, indolyl, piperidinyl, tetrahydronaphthyl, naphthyl; 2,3-dihydro-benzo[1,4]dioxinyl, benzo[1,2,5]thiadiazolyl, pyrrolidinyl, morpholinyl, where the aryl radical may be substituted one or more times by R9;
R9 is F, Cl, Br; OH, NO₂, CF₃, OCF₃, (C₁-C₆)-alkyl, (C₁-C₆)-alkyl-OH, O-(C₁-C₆)-alkyl, S-(C₁-C₆)-alkyl, (C₁-C₄)-alkylphenyl, COOH, COO-(C₁-C₆)-alkyl;
and the physiologically tolerated salts thereof, for producing a medicament for reducing the blood glucose level.

12. The use of the compound of the formula I 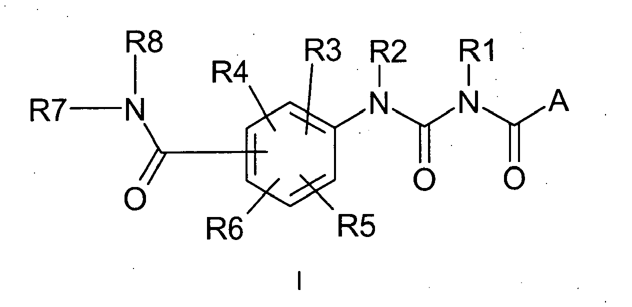 in which
A is phenyl, naphthyl, where the phenyl or naphthyl radical may be substituted up to three times by F, Cl, Br, OH, CF₃, NO₂, CN, OCF₃, O-(C₁-C₆)-alkyl, O-(C₂-C₆)-alkenyl, O-(C₂-C₆)-alkynyl, S-(C₁-C₆)-alkyl, S-(C₂-C₆)-alkenyl, S-(C₂-C₆)-alkynyl, SO-(C₁-C₆)-alkyl, SO₂-(C₁-C₆)-alkyl, SO₂-NH₂, (C₁-C₆)-alkyl, (C₂-C₆)-alkenyl, (C₂-C₆)-alkynyl, (C₃-C₇)-cycloalkyl, (C₃-C₇)-cycloalkyl-(C₁-C₄)-alkylene, (C₀-C₆)-alkylene-COOH, (C₀-C₆)-alkylene-COO-(C₁-C₇)-alkyl, (C₀-C₆)-alkylene-COO-(C₂-C₇)-alkenyl, CONH₂, CONH-(C₁-C₆)-alkyl, CON-[(C₁-C₆)-alkyl]₂, CONH-(C₃-C₆)-cycloalkyl, (C₀-C₆)-alkylene-NH₂, (C₀-C₆)-alkylene-NH-(C₁-C₆)-alkyl, (C₀-C₆)-alkylene-N-[(C₁-C₆)-alkyl]₂, NH-CO-(C₁-C₆)-alkyl, NH-CO-phenyl, NH-SO₂-phenyl, where the phenyl ring may be substituted up to twice by F, Cl, CN, OH, (C₁-C₆)-alkyl, O-(C₁-C₆)-alkyl, CF₃, OCF₃, COOH, COO-(C₁-C₆)-alkyl or CONH₂;
R1, R2 are, independently of one another, H, (C₁-C₆)-alkyl, O-(C₁-C₆)-alkyl, CO-(C₁-C₆)-alkyl, COO-(C₁-C₆)-alkyl;
R3, R4, R5, R6 are, independently of one another, H, F, Cl, Br, OH, CF₃, NO₂, CN, OCF₃, O-(C₁-C₆)-alkyl, O-(C₂-C₆)-alkenyl, O-(C₂-C₆)-alkynyl, S-(C₁-C₆)-alkyl, S-(C₂-C₆)-alkenyl, S-(C₂-C₆)-alkynyl, SO-(C₁-C₆)-alkyl, SO₂-(C₁-C₆)-alkyl, SO₂-NH₂, (C₁-C₆)-alkyl, (C₂-C₆)-alkenyl, (C₂-C₆)-alkynyl, (C₃-C₇)-cycloalkyl, (C₃-C₇)-cycloalkyl-(C₁-C₄)-alkylene, COOH, COO-(C₁-C₆)-alkyl, CO-NH₂, CO-NH-(C₁-C₆)-alkyl, CO-N-[(C₁-C₆)-alkyl]₂, CO-NH-(C₃-C₇)-cycloalkyl, NH₂, NH-(C₁-C₆)-alkyl, N-[(C₁-C₆)-alkyl]₂, NH-CO-(C₁-C₆)-alkyl, NH-CO-phenyl, NH-SO₂-phenyl, where the phenyl ring may be substituted up to twice by F, Cl, CN, OH, (C₁-C₆)-alkyl, O-(C₁-C₆)-alkyl, CF₃, OCF₃, COOH, COO-(C₁-C₆)-alkyl or CO-NH₂;
R7 is H, (C₁-C₆)-alkyl, CO(C₁-C₆)-alkyl;
R8 is H, (C₁-C₁₀)-alkyl, where alkyl may be substituted up to 3 times by OH, CF₃, CN, COOH, COO-(C₁-C₆)-alkyl, CO-NH₂, NH₂, NH-(C₁-C₆)-alkyl, N-[(C₁-C₆)-alkyl]₂, NCO-(C₁-C₆)-alkyl, NCOO-(C₁-C₆)-alkyl, NCOO-(C₁-C₆)-alkenyl, NCOO-(C₁-C₆)-alkynyl or NCOO-(C₁-C₄)-alkylene-(C₆-C₁₀)-aryl;
(CH₂)ₘ-aryl, where m can be 0-6, and aryl can be phenyl, O-phenyl, CO-phenyl, benzo[1,3]dioxolyl, heterocycloalkyl, pyridyl, indolyl, piperidinyl, tetrahydronaphthyl, naphthyl, 2,3-dihydro-benzo[1,4]dioxinyl, benzo[1,2,5]thiadiazolyl, pyrrolidinyl, morpholinyl, where the aryl radical may be substituted one or more times by R9;
R9 is F, Cl, Br; OH, NO₂, CF₃, OCF₃, (C₁-C₆)-alkyl, (C₁-C₆)-alkyl-OH, O-(C₁-C₆)-alkyl, S-(C₁-C₆)-alkyl, (C₁-C₄)-alkylphenyl, COOH, COO-(C₁-C₆)-alkyl;
and the physiologically tolerated salts thereof, for producing a medicament for treating type II diabetes.

## Revendications

1. Composés de formule I 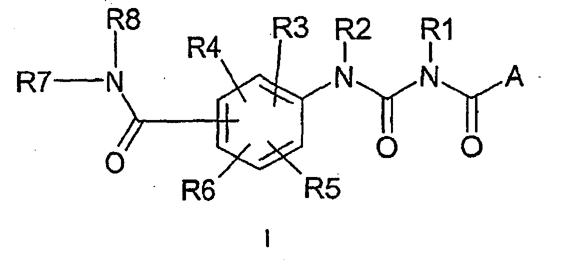 dans laquelle
A signifie phényle, naphtyle, où le radical phényle ou naphtyle peut être jusqu'à trisubstitué par F, Cl, Br, OH, CF₃, NO₂, CN, OCF₃, O-(C₁-C₆)-alkyle, O-(C₂-C₆) -alcényle, O-(C₂-C₆) -alcynyle, S-(C₁-C₆)-alkyle, S-(C₂-C₆)-alcényle, S-(C₂-C₆)-alcynyle, SO-(C₁-C₆)-alkyle, SO₂-(C₁-C₆)-alkyle, SO₂-NH₂, (C₁-C₆)-alkyle, (C₂-C₆)-alcényle, (C₂-C₆)-alcynyle, (C₃-C₇)-cycloalkyle, (C₃-C₇)-cycloalkyl-(C₁-C₄)-alkylène, (C₀-C₆) -alkylène-COOH, (C₀-C₆) -alkylène-COO- (C₁-C₇)-alkyle, (C₀-C₆)-alkylène-COO-(C₂-C₇)-alcényle, CONH₂, CONH- (C₁-C₆) -alkyle, CON- [(C₁-C₆) -alkyle]₂, CONH= (C₃-C₆) -cycloalkyle, (C₀-C₆)-alkylène-NH₂, (C₀-C₆) -alkylène-NH- (C₂-C₆) -alkyle, (C₀-C₆) -alkylène-N-[(C₁-C₆)-alkyle]₂, NH-CO- (C₁-C₆) -alkyle, NH-CO-phényle, NH-SO₂-phényle, où le cycle phényle peut être jusqu'à disubstitué par F, Cl, CN, OH, (C₁-C₆)-alkyle, O-(C₁-C₆)-alkyle, CF₃, OCF₃, COOH, COO-(C₁-C₆)-alkyle ou CONH₂ ;
R1, R2 signifient, indépendamment l'un de l'autre H, (C₁-C₆)-alkyle, O-(C₁-C₆) alkyle, CO- (C₁-C₆) alkyle, COO-(C₁-C₆)alkyle ;
R3, R4, R5, R6 signifient indépendamment l'un de l'autre H, F, Cl, Br, OH, CF₃, NO₂, CN, OCF₃, O-(C₁-C₆)-alkyle, O-(C₂-C₆)-alcényle, O-(C₂-C₆)-alcynyle, S-(C₁-C₆) -alkyle, S-(C₂-C₆) -alcényle, S-(C₂-C₆)-alcynyle, SO- (C₁-C₆)-alkyle, SO₂-(C₁-C₆)-alkyle, SO₂-NH₂, (C₁-C₆)-alkyle, (C₂-C₆)-alcényle, (C₂-C₆)-alcynyle, (C₃-C₇)-cycloalkyle, (C₃-C₇)-cycloalkyl-(C₁-C₄)-alkylène, COOH, COO-(C₁-C₆)-alkyle, CO-NH₂, CO-NH-(C₁-C₆)-alkyle, CO-N-[(C₁-C₆)-alkyle]₂, CO-NH-(C₃-C₇)-cycloalkyle, NH₂, NH-(C₁-C₆)-alkyle, N-[(C₁-C₆)-alkyle]₂, NH-CO-(C₁-C₆)-alkyle, NH-CO-phényle, NH-SO₂-phényle, où le cycle phényle peut être jusqu'à disubstitué par F, Cl, CN, OH, (C₁-C₆)-alkyle, O-(C₁-C₆)-alkyle, CF₃, OCF₃, COOH, COO-(C₁-C₆)-alkyle ou CO-NH₂ ;
R7 signifie H, (C₁-C₆)-alkyle, CO(C₁-C₆)-alkyle ;
R8 signifie H, (C₁-C₁₀)-alkyle, où alkyle peut être jusqu'à trisubstitué par OH, CF₃, CN, COOH, COO-(C₁-C₆)-alkyle, CO-NH₂, NH₂, NH-(C₁-C₆)-alkyle, N-[(C₁-C₆)-alkyle]₂, NCO- (C₁-C₆)-alkyle, NCOO- (C₁-C₆)-alkyle, NCOO-(C₁-C₆)-alcényle, NCOO-(C₁-C₆)-alcynyle ou NCOO-(C₁-C₄)-alkylène-(C₆-C₁₀)-aryle;
(CH₂)ₘ-aryle, où m peut être = 0-6 et aryle peut représenter phényle, O-phényle, CO-phényle, benzo[1,3]dioxolyle, hétérocycloalkyle, pyridyle, indolyle, pipéridinyle, tétrahydronaphtyle, naphtyle, 2,3-dihydrobenzo[1,4]dioxinyle, benzo[1,2,5]thiadiazolyle, pyrrolidinyle, morpholinyle et où le radical aryle peut être monosubstitué ou polysubstitué par R9 ;
R9 signifie F, Cl, Br, OH, NO₂, CF₃, OCF₃, (C₁-C₆)-alkyle, (C₁-C₆)-alkyl-OH, O-(C₁-C₆)-alkyle, S-(C₁-C₆)-alkyle, (C₁-C₄)-alkyl-phényle, COOH, COO-(C₁-C₆)-alkyle ;
ainsi que leurs sels physiologiquement acceptables.

2. Composés de formule I, selon la revendication 1, **caractérisés en ce que**
A signifie phényle, où le radical phényle peut être jusqu'à trisubstitué par F, Cl, Br ;
R1, R2 signifient H ;
R3, R4, R5, R6 signifient, indépendamment l'un de l'autre, H, F, Cl, Br, NO₂, O-(C₁-C₆)-alkyle, (C₁-C₆)-alkyle ;
R7 signifie H ; CH₃
R8 signifie H, (C₁-C₁₀)-alkyle, où alkyle peut être jusqu'à trisubstitué par OH, CF₃, CN, COOH, COO-(C₁-C₆) -alkyle, CO-NH₂, NH₂, NH- (C₁-C₆) -alkyle, N-[(C₁-C₆)-alkyle]₂, NCO- (C₁-C₆)-alkyle, NCOO-(C₁-C₆)-alkyle, NCOO-(C₁-C₆)-alcényle, NCOO-(C₁-C₆)-alcynyle ou NCOO-(C₁-C₄)-alkylène-(C₆-C₁₀)-aryle ;
(CH₂)ₘ-aryle, où m peut être = 0-6 et aryle peut représenter phényle, O-phényle, CO-phényle, benzo[1,3]dioxolyle, hétérocycloalkyle, pyridyle, indolyle, pipéridinyle, tétrahydronaphtyle, naphtyle, 2,3-dihydrobenzo[1,4]dioxinyle, benzo[1,2,5]thiadiazolyle, pyrrolidinyle, morpholinyle et où le radical aryle peut être monosubstitué ou polysubstitué par R9 ;
R9 signifie F, Cl, Br, OH, NO₂, CF₃, OCF₃, (C₁-C₆)-alkyle, (C₁-C₆) -alkyl-OH, O-(C₁-C₆)-alkyle, S-(C₁-C₆)-alkyle, (C₁-C₄)-alkyl-phényle, COOH, COO-(C₁-C₆)-alkyle ;
ainsi que leurs sels physiologiquement acceptables.

3. Composés de formule I, selon la revendication 1, **caractérisés en ce que**
A signifie phényle, où le radical phényle peut être jusqu'à trisubstitué par F, Cl, Br ;
R1, R2 signifient H ;
R3, R4, R5, R6 signifient, indépendamment l'un de l'autre, H, F, Cl, Br, NO₂, O-(C₁-C₆)-alkyle, (C₁-C₆)-alkyle ;
R7 signifie H ; CH₃
R8 signifie (C₁-C₁₀)-alkyle, où alkyle peut être jusqu'à trisubstitué par OH, CF₃, CN, COOH, COO-(C₁-C₆)-alkyle, CO-NH₂, NH₂, NH-(C₁-C₆)-alkyle, N-[(C₁-C₆)-alkyle]₂, NCO-(C₁-C₆)-alkyle, NCOO-(C₁-C₆)-alkyle, NCOO-(C₁-C₆)-alcényle, NCOO-(C₁-C₆)-alcynyle ou NCOO-(C₁-C₄)-alkylène-(C₆-C₁₀)-aryle ;
(CH₂)ₘ-aryle, où m peut être = 0-6 et aryle peut représenter phényle, O-phényle, CO-phényle, benzo[1,3]dioxolyle, hétérocycloalkyle, pyridyle, indolyle, pipéridinyle, tétrahydronaphtyle, naphtyle, 2,3-dihydrobenzo[1,4]dioxinyle, benzo[1,2,5]thiadiazolyle, pyrrolidinyle, morpholinyle et où le radical aryle peut être monosubstitué ou polysubstitué par R9 ;
R9 signifie F, Cl, Br, OH, NO₂, CF₃, OCF₃, (C₁-C₆)-alkyle, (C₁-C₆)-alkyl-OH, O-(C₁-C₆)-alkyle, S-(C₁-C₆)-alkyle, (C₁-C₄)-alkyl-phényle, COOH, COO-(C₁-C₆)-alkyle ;
ainsi que leurs sels physiologiquement acceptables.

4. Médicament contenant un ou plusieurs des composés selon l'une ou plusieurs des revendications 1 à 3.

5. Médicament contenant un ou plusieurs des composés selon l'une ou plusieurs des revendications 1 à 3 et une ou plusieurs substances actives diminuant la glycémie.

6. Utilisation des composés selon l'une ou plusieurs des revendications 1 à 3 pour la préparation d'un médicament destiné au traitement du diabète de type II.

7. Utilisation des composés selon l'une ou plusieurs des revendications 1 à 3 pour la préparation d'un médicament destiné à abaisser la glycémie.

8. Utilisation des composés selon l'une ou plusieurs des revendications 1 à 3 en combinaison avec au moins une autre substance active diminuant la glycémie pour la préparation d'un médicament destiné au traitement du diabète de type II.

9. Utilisation des composés selon l'une ou plusieurs des revendications 1 à 3 en combinaison avec au moins une autre substance active diminuant la glycémie pour la préparation d'un médicament destiné à la diminution de la glycémie.

10. Procédé pour la préparation d'un médicament contenant un ou plusieurs des composés selon l'une ou plusieurs des revendications 1 à 3, **caractérisé en ce que** la substance active est mélangée avec un support pharmaceutiquement approprié et ce mélange est amené dans une forme appropriée pour l'administration.

11. Utilisation du composé de formule I 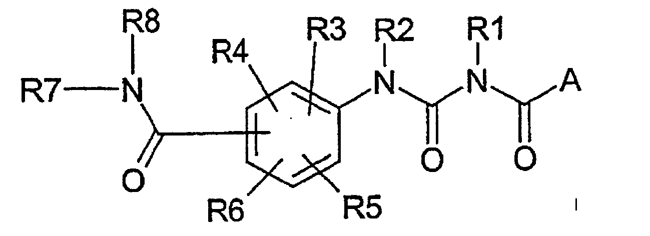 dans laquelle
A signifie phényle, naphtyle, où le radical phényle ou naphtyle peut être jusqu'à trisubstitué par F, Cl, Br, OH, CF₃, NO₂, CN, OCF₃, O-(C₁-C₆)-alkyle, O-(C₂-C₆) -alcényle, 0- (C₂-C₆) -alcynyle, S-(C₁-C₆)-alkyle, S-(C₂-C₆)-alcényle, S-(C₂-C₆)-alcynyle, SO-(C₁-C₆)-alkyle, SO₂-(C₁-C₆)-alkyle, SO₂-NH₂, (C₁-C₆)-alkyle, (C₂-C₆)-alcényle, (C₂-C₆)-alcynyle, (C₃-C₇)-cycloalkyle, (C₃-C₇) -cycloalkyl- (C₁-C₄)-alkylène, (C₀-C₆)-alkylène-COOH, (C₀-C₆)-alkylène-COO- (C₁-C₇)-alkyle, (C₀-C₆)-alkylène-COO-(C₂-C₇)-alcényle, CONH₂, CONH-(C₁-C₆)-alkyle, CON-[(C₁-C₆)-alkyle]₂, CONH-(C₃-C₆)-cycloalkyle, (C_{O}-C₆)-alkylène-NH₂, (C₀-C₆)-alkylène-NH-(C₁-C₆)-alkyle, (C₀-C₆)-alkylène-N-[(C₁-C₆)-alkyle]₂, NH-CO-(C₁-C₆)-alkyle, NH-CO-phényle, NH-SO₂-phényle, où le cycle phényle peut être jusqu'à disubstitué par F, Cl, CN, OH, (C₁-C₆)-alkyle, O-(C₁-C₆)-alkyle, CF₃, OCF₃, COOH, COO-(C₁-C₆)-alkyle ou CONH₂ ;
R1, R2 signifient, indépendamment l'un de l'autre H, (C₁-C₆)-alkyle, O-(C₁-C₆) alkyle, CO-(C₁-C₆) alkyle, COO-(C₁-C₆) alkyle ;
R3, R4, R5, R6 signifient indépendamment l'un de l'autre H, F, Cl, Br, OH, CF₃, NO₂, CN, OCF₃, O-(C₁-C₆)-alkyle, O-(C₂-C₆)-alcényle, O-(C₂-C₆)-alcynyle, S-(C₁-C₆)-alkyle, S-(C₂-C₆)-alcényle, S-(C₂-C₆)-alcynyle, SO-(C₁-C₆) -alkyle, SO₂-(C₁-C₆)-alkyle, SO₂-NH₂, (C₁-C₆)-alkyle, (C₂-C₆)-alcényle, (C₂-C₆)-alcynyle, (C₃-C₇)-cycloalkyle, (C₃-C₇)-cycloalkyl-(C₁-C₄)-alkylène, COOH, COO-(C₁-C₆)-alkyle, CO-NH₂, CO-NH-(C₁-C₆)-alkyle, CO-N-(C₁-C₆)-alkyle]₂, CO-NH-(C₃-C₇)-cycloalkyle, NH₂, NH-(C₁-C₆)-alkyle, N-[(C₁-C₆)-alkyle]₂, NH-CO-(C₁-C₆)-alkyle, NH-CO-phényle, NH-SO₂-phényle, où le cycle phényle peut être jusqu'à disubstitué par F, Cl, CN, OH, (C₁-C₆)-alkyle, O-(C₁-C₆) -alkyle, CF₃, OCF₃, COOH, COO-(C₁-C₆) -alkyle ou CO-NH₂ ;
R7 signifie H, (C₁-C₆)-alkyle, CO(C₁-C₆)-alkyle ;
R8 signifie H, (C₁-C₁₀)-alkyle, où alkyle peut être jusqu'à trisubstitué par OH, CF₃, CN, COOH, COO-(C₁-C₆) -alkyle, CO-NH₂, NH₂, NH- (C₁-C₆) -alkyle, N-[(C₁-C₆)-alkyle]₂, NCO- (C₁-C₆)-alkyle, NCOO-(C₁-C₆)-alkyle, NCOO-(C₁-C₆)-alcényle, NCOO-(C₁-C₆)-alcynyle ou NCOO-(C₁-C₄) -alkylène-(C₆-C₁₀)-aryle ;
(CH₂)ₘ-aryle, où m peut être = 0-6 et aryle peut représenter phényle, O-phényle, CO-phényle, benzo[1,3]dioxolyle, hétérocycloalkyle, pyridyle, indolyle, pipéridinyle, tétrahydronaphtyle, naphtyle, 2,3-dihydrobenzo[1,4]dioxinyle, benzo[1,2,5]thiadiazolyle, pyrrolidinyle, morpholinyle et où le radical aryle peut être monosubstitué ou polysubstitué par R9 ;
R9 signifie F, Cl, Br, OH, NO₂, CF₃, OCF₃, (C₁-C₆)-alkyle, (C₁-C₆) -alkyl-OH, O-(C₁-C₆)-alkyle, S-(C₁-C₆)-alkyle, (C₁-C₄)-alkyl-phényle, COOH, COO-(C₁-C₆)-alkyle ;
ainsi que leurs sels physiologiquement acceptables pour la préparation d'un médicament pour la diminution de la glycémie.

12. Utilisation du composé de formule I 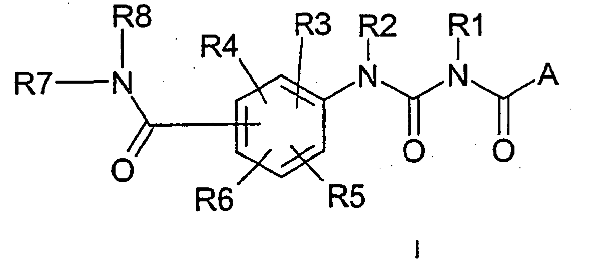 dans laquelle
A signifie phényle, naphtyle, où le radical phényle ou naphtyle peut être jusqu'à trisubstitué par F, Cl, Br, OH, CF₃, NO₂, CN, OCF₃, O-(C₁-C₆)-alkyle, O-(C₂-C₆)-alcényle, O-(C₂-C₆)-alcynyle, S-(C₁-C₆)-alkyle, S-(C₂-C₆) -alcényle, S-(C₂-C₆)-alcynyle, SO-(C₁-C₆)-alkyle, SO₂-(C₁-C₆) -alkyle, SO₂-NH₂, (C₁-C₆)-alkyle, (C₂-C₆)-alcényle, (C₂-C₆)-alcynyle, (C₃-C₇)-cycloalkyle, (C₃-C₇) -cycloalkyl- (C₁-C₄) -alkylène, (C₀-C₆)-alkylène-COOH, (C₀-C₆)-alkylène-COO-(C₁-C₇)-alkyle, (C₀-C₆)-alkylène-COO-(C₂-C₇)-alcényle, CONH₂, CONH-(C₁-C₆)-alkyle, CON-[(C₁-C₆)-alkyle]₂, CONH- (C₃-C₆) -cycloalkyle, (C₀-C₆)-alkylène-NH₂, (C₀-C₆)-alkylène-NH-(C₁-C₆)-alkyle, (C₀-C₆)-alkylène-N-[(C₁-C₆)-alkyle]₂, NH-CO-(C₁-C₆)-alkyle, NH-CO-phényle, NH-SO₂-phényle, où le cycle phényle peut être jusqu'à disubstitué par F, Cl, CN, OH, (C₁-C₆)-alkyle, O-(C₁-C₆)-alkyle, CF₃, OCF₃, COOH, COO-(C₁-C₆)-alkyle ou CONH₂;
R1, R2 signifient, indépendamment l'un de l'autre H, (C₁-C₆)-alkyle, O-(C₁-C₆) alkyle, CO-(C₁-C₆) alkyle, COO-(C₁-C₆)alkyle;
R3, R4, R5, R6 signifient indépendamment l'un de l'autre H, F, Cl, Br, OH, CF₃, NO₂, CN, OCF₃, O-(C₁-C₆)-alkyle, O-(C₂-C₆)-alcényle, O-(C₂-C₆)-alcynyle, S-(C₁-C₆)-alkyle, S-(C₂-C₆)-alcényle, S-(C₂-C₆)-alcynyle, SO- (C₁-C₆)-alkyle, SO₂-(C₁-C₆)-alkyle, SO₂-NH₂, (C₁-C₆)-alkyle, (C₂-C₆)-alcényle, (C₂-C₆)-alcynyle, (C₃-C₇)-cycloalkyle, (C₃-C₇)-cycloalkyl-(C₁-C₄)-alkylène, COOH, COO-(C₁-C₆)-alkyle, CO-NH₂, CO-NH-(C₁-C₆)-alkyle, CO-N- [(C₁-C₆)-alkyle]₂, CO-NH-(C₃-C₇)-cycloalkyle, NH₂, NH-(C₁-C₆)-alkyle, N-[(C₁-C₆)-alkyle]₂, NH-CO-(C₁-C₆)-alkyle, NH-CO-phényle, NH-SO₂-phényle, où le cycle phényle peut être jusqu'à disubstitué par F, Cl, CN, OH, (C₁-C₆)-alkyle, O-(C₁-C₆)-alkyle, CF₃, OCF₃, COOH, COO-(C₁-C₆)-alkyle ou CO-NH₂ ;
R7 signifie H, (C₁-C₆)-alkyle, CO(C₁-C₆)-alkyle ;
R8 signifie H, (C₁-C₁₀)-alkyle, où alkyle peut être jusqu'à trisubstitué par OH, CF₃, CN, COOH, COO-(C₁-C₆)-alkyle, CO-NH₂, NH₂, NH-(C₁-C₆)-alkyle, N-[(C₁-C₆)-alkyle]₂, NCO-(C₁-C₆)-alkyle, NCOO-(C₁-C₆)-alkyle, NCOO-(C₁-C₆)-alcényle, NCOO-(C₁-C₆)-alcynyle ou NCOO-(C₁-C₄)-alkylène-(C₆-C₁₀)-aryle ;
(CH₂)ₘ-aryle, où m peut être = 0-6 et aryle peut représenter phényle, O-phényle, CO-phényle, benzo[1,3]dioxolyle, hétérocycloalkyle, pyridyle, indolyle, pipéridinyle, tétrahydronaphtyle, naphtyle, 2,3-dihydrobenzo[1,4]dioxinyle, benzo[1,2,5]thiadiazolyle, pyrrolidinyle, morpholinyle et où le radical aryle peut être monosubstitué ou polysubstitué par R9 ;
R9 signifie F, Cl, Br, OH, NO₂, CF₃, OCF₃, (C₁-C₆)-alkyle, (C₁-C₆)-alkyl-OH, 0- (C₁-C₆) -alkyle, S-(C₁-C₆)-alkyle, (C₁-C₄)-alkyl-phényle, COOH, COO-(C₁-C₆)-alkyle ;
ainsi que leurs sels physiologiquement acceptables pour la préparation d'un médicament destiné au traitement du diabète de type II.
